# EUROPEAN PATENT SPECIFICATION

(11) **EP 2 074 971 B1**
(45) Date of publication and mention of the grant of the patent: **13.04.2011**
(21) Application number: 08254159.0
(22) Date of filing: 24.12.2008
(51) Int. Cl.: A61F 5/00, A61F 2/00

(54) **Fluid logic for regulating restriction devices**
Fluidik zur Regelung von Drosselungsvorrichtungen
Logique à fluide pour la régulation de dispositifs de restriction

(30) Priority: 27.12.2007 US 965334
(43) Date of publication of application: 01.07.2009
(73) Proprietor: Ethicon Endo-Surgery, Inc., Cincinnati, OH 45242 (US)
(72) Inventor: Coe, Jonathan A., Cincinnati Ohio 45236 (US); Moore, Kyle P., Mason Ohio 45040 (US); Zwolinski, Andrew M., Cincinnati Ohio 45226 (US); Adams, Thomas E., Maineville Ohio 45039 (US); Simms, Robert Jason, Liberty Township Ohio 45044 (US); Stegeman, JoAnn M., Skokie Illinois 60076 (US); Widenhouse, Christopher W., Clarksville Ohio 45113 (US); Ezolino, Juan S., Weston Florida 33326 (US); Overymyer, Mark D., Grandville Michigan 49418 (US)
(74) Representative: Tunstall, Christopher Stephen

(56) References cited:
- US-A1- 2007 156 013
- US-B2- 7 217 237

## Description

### FIELD

The present invention relates to devices for forming a restriction in a pathway, and in particular to fluid logic systems for controlling fluid pressure in a restriction system.

### BACKGROUND

Obesity is becoming a growing concern, particularly in the United States, as the number of obese people continues to increase, and more is learned about the negative health effects of obesity. Morbid obesity, in which a person is 100 pounds or more over ideal body weight, in particular poses significant risks for severe health problems. Accordingly, a great deal of attention is being focused on treating obese patients. One method of treating morbid obesity has been to place a restriction device, such as an elongated band, about the upper portion of the stomach. Gastric bands have typically comprised a fluid-filled elastomeric balloon with fixed endpoints that encircles the stomach just inferior to the esophageal-gastric junction to form a small gastric pouch above the band and a reduced stoma opening in the stomach. When fluid is infused into the balloon, the band expands against the stomach creating a food intake restriction or stoma in the stomach. To decrease this restriction, fluid is removed from the band. The effect of the band is to reduce the available stomach volume and thus the amount of food that can be consumed before becoming "full."

With each of the above-described food restriction devices, safe, effective treatment requires that the device be regularly monitored and adjusted to vary the degree of restriction applied to the stomach. With banding devices, the gastric pouch above the band will substantially increase in size following the initial implantation. Accordingly, the stoma opening in the stomach must initially be made large enough to enable the patient to receive adequate nutrition while the stomach adapts to the banding device. As the gastric pouch increases in size, the band may be adjusted to vary the stoma size. In addition, it is desirable to vary the stoma size in order to accommodate changes in the patient's body or treatment regime, or in a more urgent case, to relieve an obstruction or severe esophageal dilatation. Traditionally, adjusting a hydraulic gastric band requires a scheduled clinician visit during which a huber needle and syringe are used to permeate the patient's skin and add or remove fluid from the balloon. More recently, implantable pumps have been developed which enable non-invasive adjustments of the band. An external programmer communicates with the implanted pump using telemetry to control the pump. During a scheduled visit, a physician places a hand-held portion of the programmer near the gastric implant and transmits power and command signals to the implant. The implant in turn adjusts the fluid levels in the band and transmits a response command to the programmer.

While such techniques are successful in adjusting the band pressure, there remains a need for improved techniques. Conventional hydraulic gastric banding devices exert a continuous restricting force on the stomach to reduce the size of the upper stomach and to restrict the passage of food from the upper to the lower stomach. However, side effects and complications of conventional gastric banding devices include erosion of the exterior stomach tissue resulting from the constant pressure of the band on the exterior stomach. In addition, hydraulic bands do not offer stable banding over time. Liquid within the bands diffuses slowly through the elastomer. Hydraulic bands therefore cannot guarantee the optimal configuration of the band over time. Multiple adjustments to maintain the optimal configuration of the band are required, increasing the cost and the number of medical visits. Also, adjustment of the band requires puncture of the patient's skin, resulting in discomfort for the patient and an increased risk of infection.

US200710156013A1 describes a self-regulating gastric band apparatus. The band adjustment assembly includes a pump assembly connected to a controller and an expandable portion containing a volume of fluid.

US7217237B2 describes a closing system, said to be suitable for the selective closing and opening of a tubular body organ, specifically for electronic control of an artificial fine-sensory sphincter implant.

Accordingly, there remains a need for methods and devices for regulating a hydraulic restriction system.

### SUMMARY

Systems are generally provided for automatically regulating a restriction in a pathway. In one embodiment, a self-regulating restriction system is provided and includes both a restriction device configured to receive fluid to form a restriction in a pathway and a fluid logic system coupled to the restriction device and configured to regulate an amount of fluid in the restriction device in response to a fluid pressure to thereby control the size of the pathway. The fluid logic system can include one or more fluid reservoirs and one or more valves coupled between the fluid reservoir(s) and the restriction device. In one exemplary embodiment, the fluid logic system defines one or more pre-set pressure limits that allow valves to be configured such that when a pressure of fluid in the restriction device is less than or greater than the pre-set pressure limit(s) the valve(s) can move from a closed to an opened position to allow fluid to flow between the fluid reservoir(s) and the restriction device. More specifically, pre-set pressure limits can include a minimum pre-set pressure limit that allows the valve(s) to be configured such that when the pressure of fluid in the restriction device is less than the minimum pre-set pressure limit the valve(s) can open to allow fluid flow from the fluid reservoir(s), which in a preferred embodiment is a high pressure fluid reservoir, to the restriction device. When the pressure of fluid in the restriction device is raised to be equal to and/or greater than the minimum pre-set pressure limit, the valve(s) can be configured to close to stop fluid flow from the fluid reservoir(s) to the restriction device. Likewise, pre-set pressure limits can also include a maximum pre-set pressure limit that allows the valve(s) to be configured such that when the pressure of fluid in the restriction device is greater than the maximum pre-set pressure limit the valve(s) can open to allow fluid flow from the restriction device to the fluid reservoir(s), which in a preferred embodiment is a low pressure fluid reservoir. When the pressure of fluid in the restriction device is lowered to be equal to and/or less than the maximum pre-set pressure, the valve(s) can be configured to close to stop fluid flow from the restriction device to the fluid reservoir(s). Embodiments that include any combination of a minimum and maximum pre-set pressure limit and a high and low pressure fluid reservoir can be used in a fluid logic system, and in at least one embodiment of a fluid logic system, minimum and maximum pre-set pressure limits can be used with high and low pressure fluid reservoirs. Further, one or more valves can be configured to be operational with any such system that includes any combination of the minimum and maximum pre-set pressure limits and the high and low pressure fluid reservoirs, including an embodiment that has both minimum and maximum pre-set pressure limits and high and low pressure fluid reservoirs. The pre-set pressure limit(s) of the fluid logic system can also be fixed or adjustable, for instance by adjusting a pressure of fluid in the fluid reservoir(s) or by adjusting one or more parameters of the valve(s).

While the fluid logic system can include one or more valves and reservoirs a logic valve is used to regulate the amount of fluid in the restriction device in response to a fluid pressure. More particularly, the logic valve is configured to regulate fluid flow in response to a pressure of fluid in the restriction device. The logic valve is coupled to a biasing mechanism that is effective to apply a biasing force to the logic valve that counteracts a force applied to the logic valve by a pressure of fluid in the restriction device. In an exemplary embodiment, the biasing mechanism can be adjustable to allow the biasing force to be adjusted, which in turn allows the one or more pre-set pressure limits of the fluid logic system to be set and/or adjusted. While logic valves can be configured in a variety of ways, in one embodiment the valve includes a first port in fluid communication with the fluid reservoir(s) and a second port in fluid communication with the restriction device. The logic valve can also include one or more seals configured to regulate fluid flow between the first and second ports to thereby regulate fluid flow between the fluid reservoir(s) and the restriction device. A third port can also be included, for instance by placing it in fluid communication with the fluid reservoir(s), and further, the seal(s) can be configured to regulate flow between the second port and the third port to thereby regulate fluid flow between the fluid reservoir(s) and the restriction device. More particularly, the logic valve can be configured to regulate fluid flow between the first, second, and third ports in response to a pressure of fluid in the restriction device. In one exemplary embodiment, the first port can be in communication with a high pressure fluid reservoir and the third port can be in communication with a low pressure fluid reservoir.

It is also disclosed that another type of valve that can be used in the fluid logic system is a regulator valve. The regulator valve can include a bi-stable beam that is effective to selectively open and close the regulator valve to regulate fluid flow in response to a pressure of fluid in the restriction device. More particularly, the bi-stable bearn can be configured to buckle when a force is applied to the beam, which in turn can cause the regulator valve to open to allow the flow of fluid between the fluid reservoir(s) and the restriction device. A biasing mechanism can be coupled to the bi-stable beam and effective to apply a biasing force to the beam to direct it toward a buckled configuration, while a force applied to the beam by a pressure of fluid in the restriction device can be effective to counteract the biasing force. In another disclosure of a regulator valve, the regulator valve can include a gate movable between an opened position, in which fluid can flow from the fluid reservoir(s) to the restriction device, and a closed position, in which fluid can be prevented from flowing between the fluid reservoir(s) and the restriction device. The gate can be movable based on a variety of forces that are applied to the gate, but in an exemplary disclosure a biasing mechanism applies a biasing force to the gate to bias the gate toward the opened position and a force applied by a pressure of fluid in the restriction device is effective to counteract the biasing force of the biasing mechanism to move the gate toward the closed position. Further, the biasing mechanism can be adjustable to allow the biasing force to be adjusted, which in turn allows the one or more pre-set pressure limits of the fluid logic system to be set and/or adjusted.

In another disclosure, the one or more valves of the fluid logic system can be a check valve. The check valve can be configured to have a cracking pressure, which is a pressure at which the valve is configured to open or close to allow or prevent fluid flow between the reservoir(s) and the restriction device in response to a particular parameter, for example a pressure of fluid in the restriction device. In some versions, the check valve can have multiple cracking pressures. In an exemplary disclosure, the fluid logic system includes two fluid reservoirs and two check valves, and further, the first check valve is coupled to a high pressure fluid reservoir and the second check valve is coupled to a low pressure fluid reservoir. The first and second check valves can have separate cracking pressures. When a pressure of fluid in the restriction device is less than the cracking pressure of the first check valve, the first check valve can be configured to open and release fluid from the high pressure fluid reservoir into the restriction device. When a pressure of fluid in the restriction device increases to an amount equal to and/or greater than the cracking pressure of the first check valve, the check valve can be configured to close to stop fluid flow from the high pressure fluid reservoir to the restriction device. Likewise, when a pressure of fluid in the restriction device is greater than the cracking pressure of the second check valve, the second check valve can be configured to open and release fluid from the restriction device to the low pressure fluid reservoir. When a pressure of fluid in the restriction device decreases to an amount equal to and/or less than the cracking pressure of the second check valve, the check valve can be configured to close to stop fluid flow from the restriction device to the low pressure fluid reservoir. In an exemplary disclosure, the check valve can be adjustable. For example, the cracking pressure of the check valve can be adjusted, for instance, by adjusting a pressure of fluid in the fluid reservoir(s) or by adjusting one or more parameters of the adjustable check valve. In one disclosure the check valve can be a magnetic check valve.

The fluid logic system can incorporate a variety of fluid reservoirs. In one exemplary embodiment, the fluid reservoir can be a high pressure fluid reservoir. One example of such a high pressure fluid reservoir is an osmotic pump. In another embodiment, the high pressure fluid reservoir can include a chamber containing chemical reactants and configured to react to generate a high pressure. Further, the chamber can include a port configured to allow the chemical reactants to be altered to change the high pressure output of the fluid reservoir. Various types of reactants can be used, but in one embodiment the resultant reaction is an exothermic reaction.

A method for maintaining a restriction in a pathway is also disclosure. In one exemplary embodiment, a restriction device can be implanted in a patient to form a restriction in a pathway such that the restriction in the pathway corresponds to an amount of fluid contained within the restriction device. A fluid logic system can be coupled to the restriction device and configured to regulate an amount of fluid in the restriction device in response to a fluid pressure to thereby control the size of the pathway. In one embodiment, the fluid logic system can include one or more fluid reservoirs and one or more valves coupled between the fluid reservoir(s) and the restriction device. The valves can be configured to regulate fluid flow between the reservoir(s) and the restriction device. One method for regulating the flow of fluid between the reservoir(s) and the restriction device is to configure the valve(s) to maintain a pressure of fluid in the restriction device within a pre-set pressure range. The pre-set pressure range can include a variety of different types of pressures, but in one exemplary embodiment the range includes a minimum pre-set pressure and a maximum pre-set pressure. When a pressure of fluid in the restriction device is less than the minimum pre-set pressure, the valve(s) can be configured to open to allow fluid flow from the fluid reservoir(s) to the restriction device. When a pressure of fluid in the restriction device is equal to and/or greater than the minimum pre-set pressure, the valve(s) can be configured to close to stop fluid flow from the fluid reservoir(s) to the restriction device. Likewise, when a pressure of fluid in the restriction device is greater than the maximum pre-set pressure, the valve(s) can be configured to open to allow fluid flow from the restriction device to the fluid reservoir(s). When a pressure of fluid in the restriction device is equal to and/or less than the maximum pre-set pressure, the valve(s) can be configured to close to stop fluid flow from the restriction device to the fluid reservoir(s). In one exemplary embodiment, the method can include adjusting the pre-set pressure range, for instance by adjusting a pressure of fluid in the fluid reservoir(s) or by adjusting one or more parameters of the valve(s).

### BRIEF DESCRIPTION OF THE DRAWINGS

The invention will be more fully understood from the following detailed description taken in conjunction with the accompanying drawings, in which:

FIG. 1 is a schematic diagram illustrating one exemplary embodiment of a restriction system having a fluid logic system for controlling fluid flow through the system;

FIG. 2A is an illustration of the gastric restriction system of FIG. 1 implanted to form a restriction in a patient's stomach;

FIG. 2B is a perspective view of a gastric restriction device and port of the gastric restriction system of FIGS. 1 and 2A;

FIG. 3 is a schematic diagram illustrating one embodiment of a fluid logic system having a logic valve for regulating fluid flow;

FIG. 4A is a perspective, partially transparent view of one exemplary embodiment of a logic valve for use with the fluid logic system of FIG. 3;

FIG. 4B is a cross-sectional view of the logic valve of FIG. 4A, showing the valve in a first, open position;

FIG. 4C is a cross-sectional view of the logic valve of FIG. 4A, showing the valve in a closed position;

FIG. 4D is a cross-sectional view of the logic valve of FIG. 4A, showing the valve in a second, open position;

FIG. 5 is a schematic diagram illustrating another embodiment not of the present invention of a fluid logic system having a regulator valve for regulating fluid flow;

FIG. 6A is a cross-sectional view of one exemplary embodiment not of the present invention of a regulator valve for use with the fluid logic system of FIG. 5;

FIG. 6B is a perspective view of a portion of the regulator valve of FIG. 6A;

FIG. 7 is a cross-sectional view of another exemplary embodiment of a regulator valve for use with the fluid logic system of FIG. 5;

FIG. 8 is a schematic diagram illustrating yet another embodiment of a fluid logic system not of the present invention having a check valve for regulating fluid flow;

FIG. 9A is a cross-sectional view of one exemplary embodiment of a check valve for use with a fluid logic system not of the present invention in a closed position.

FIG. 9B is a cross-sectional view of the check valve of FIG. 9A in an opened position;

FIG. 9C is a cross-sectional view of the check valve of FIG. 9A having an adjustment mechanism coupled thereto;

FIG. 10A is a cross-sectional view of yet another exemplary embodiment of a check valve for use with a fluid logic system not of the present invention;

FIG. 10B is a cross-sectional view of a check valve for use with a fluid logic system according to another embodiment not of the present invention;

FIG. 11A is a cross-sectional view of one embodiment of a high pressure fluid reservoir for use with a fluid logic system;

FIG. 11B is a cross-sectional view of another embodiment of a high pressure fluid reservoir for use with a fluid logic system;

FIG. 11C is a cross-sectional view of yet another embodiment of a high pressure fluid reservoir for use with a fluid logic system; and

FIG. 12 is a perspective view of another embodiment of a high pressure fluid reservoir for use with a fluid logic system.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

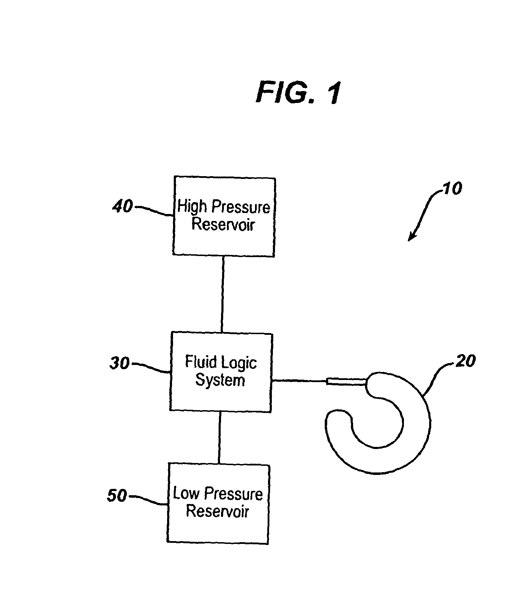

The present invention generally provides devices for regulating a restriction system. In one exemplary embodiment, as shown in FIG. 1, a restriction system 10 is provided having a restriction device 20 configured to receive fluid and to form a restriction in a pathway corresponding to an amount of fluid contained therein, at least one fluid reservoir, and a fluid logic system 30 coupled between the restriction device 20 and the at least one fluid reservoir. In the illustrated embodiment, the system 10 includes a high pressure fluid reservoir 40 and a low pressure fluid reservoir 50, however the system can include any number of reservoirs having any pressure as may be needed, as will be discussed in more detail below. In use, the fluid logic system 30 is configured to regulate an amount of fluid in the restriction device in response to a fluid pressure acting thereon. The use of a fluid logic system to regulate a pressure of fluid in the restriction device 20 is particularly advantageous as it allows the system to be self-regulating, without the need for adjustments over time as changes occur in the patient. The use of a fluid logic system is also particularly advantageous as it can mechanically regulate the pressure of the restriction device without the use of any electrical components that may need to be powered to operate over extended periods of time.

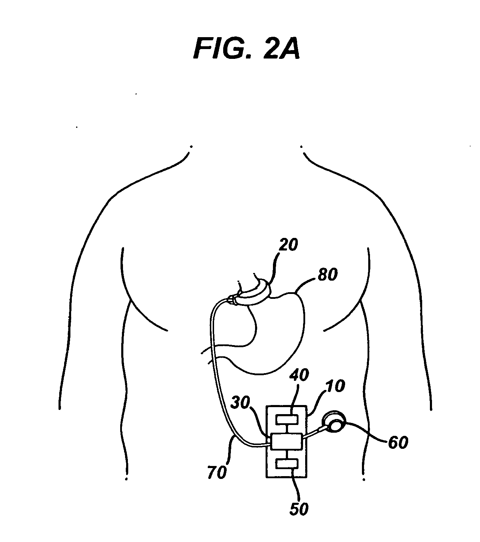

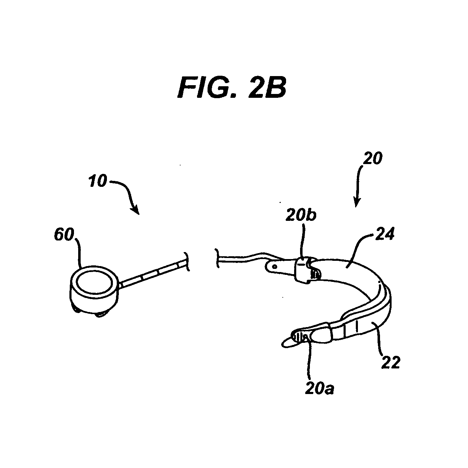

FIG. 2A illustrates the restriction system 10 implanted to form a restriction in a patient's stomach 80. In the illustrated embodiment the restriction device 20 is a gastric restriction band that is positioned around the upper portion of a patient's stomach 80, however the present invention can be used with virtually any restriction device. The illustrated restriction device 20 is shown in more detail in FIG. 2B, and as shown the restriction device 20 has a generally elongate shape with a support structure 22 having first and second opposite ends 20a, 20b that can be secured to each other. Various mating techniques can be used to secure the ends 20a, 20b to one another. In the illustrated embodiment, the ends 20a, 20b are in the form of straps that mate together, with one laying on top of the other. The gastric band 20 can also include a variable volume member, such as an inflatable balloon 24, that is disposed or formed on one side of the support structure 22, and that is configured to be positioned adjacent to tissue. The balloon 24 can contain a variable amount of fluid that causes the balloon 24 to expand or contract against the outer wall of the stomach to form an adjustable stoma for controllably restricting food intake into the stomach. In use, the gastric restriction device 20 can be applied about the gastro-esophageal junction of a patient. As shown in FIG. 2A, the restriction device 20 at least substantially encloses the upper portion of the stomach 80 near the junction with the esophagus. After the restriction device 20 is implanted, preferably in the deflated configuration wherein the restriction device 20 contains little or no fluid, the restriction device 20 can be inflated, e.g., using saline, to decrease the size of the stoma opening. A person skilled in the art will appreciate that various techniques, including those disclosed herein, can be used to initially inflate and/or adjust the restriction device 20.

A person skilled in the art will appreciate that the gastric band can have a variety of other configurations, moreover the various devices disclosed herein have equally applicability to other types of restriction devices. For example, bands are used for the treatment of fecal incontinence, as described in U.S. Pat. No. 6,461,292 . Bands can also be used to treat urinary incontinence, as described in U.S. Patent Application No. 2003/0105385 . Bands can also be used to treat heartburn and/or acid reflux, as disclosed in U.S. Pat. No. 6,470,892 . Bands can also be used to treat impotence, as described in U.S. Patent Application No. 2003/0114729.

As further shown in FIG. 2A, the fluid logic system 30, as well as any fluid reservoirs, e.g., reservoir 40 and reservoir 50, coupled thereto can also be implanted in the patient. The particular location can vary as desired by the surgeon. In use, as indicated above, the logic system 30 can be configured to regulate a pressure of fluid in the restriction device 20 to thereby increase or decrease an amount of restriction created by the restriction device 20. The fluid logic system 30 can have virtually any configuration that is effective to control fluid flow, but in certain exemplary embodiments the fluid logic system 30 has a pre-set pressure limit, or a pre-set pressure range, that the fluid logic system 30 relies on to achieve a desired pressure in the restriction device 20. For example, the fluid logic system can define at least one pre-set pressure limit, and when a pressure of fluid in the restriction device is less than or greater than the pre-set pressure limit(s) the fluid logic system can allow fluid to flow between at least one fluid reservoir 40, 50 and the restriction device 20. The system can thus control an amount of fluid added into and/or removed from the restriction device 20, thereby controlling an amount of restriction that is formed by the restriction device 20.

In one exemplary embodiment, the fluid logic system 30 can have a minimum pre-set pressure limit such that, when the pressure of the fluid in the restriction device drops below the minimum pre-set pressure limit (for example, due to patient weight loss), fluid is released from the fluid reservoir into the restriction device 20 to thereby increase the pressure in the restriction device 20 (thereby increasing the amount of restriction) until the pressure is equal to or greater than the minimum pre-set pressure limit. In such an embodiment, the fluid reservoir is preferably the high pressure fluid reservoir 40, as the high pressure will force fluid to flow into the restriction device 20. Alternatively, or in addition, the fluid logic system 30 can have a maximum pre-set pressure limit such that, when the pressure of the fluid in the restriction device 20 exceeds the maximum pre-set pressure limit (for example, due to a food blockage in the pathway), fluid is released from the restriction device 20 into the fluid reservoir to thereby decrease the pressure in the restriction device 20 (thereby decreasing the amount of restriction) until the pressure is equal to or less than the maximum pre-set pressure limit. In such an embodiment, the fluid reservoir is preferably the low pressure fluid reservoir 50, as the low pressure will allow fluid to flow from the restriction device 20 into the reservoir 50. Having a pre-set pressure limit(s) is particularly advantageous as it allows for small variations in the pressure in the restriction device 20, for example while the patient is eating, without continuously altering the fluid pressure in the restriction device, yet it is effective to maintain the pressure within a desired range to provide an amount of restriction necessary for the device to be effective.

The maximum pre-set pressure limit and the minimum pre-set pressure limit can be defined based on various parameters of the system, and one or both of the limits can be fixed or adjustable. In one exemplary embodiment, the minimum pre-set pressure limit (hereinafter Pₘᵢₙ) is defined by the difference between the fluid pressure in the high pressure fluid reservoir (hereinafter P_{H}) and a first pressure (hereinafter P₁) created by a component of the fluid logic system. In other words, Pₘᵢₙ = P_{H} - P₁. When the pressure of fluid in the restriction device (hereinafter Pᵣ) is less than Pₘᵢₙ, the fluid logic system will allow fluid flow from the high pressure fluid reservoir to the restriction device. Conversely, when Pᵣ is less than or equal to Pₘᵢₙ, the fluid logic system will prevent fluid flow from the high pressure fluid reservoir to the restriction device. In another exemplary embodiment, the maximum pre-set pressure limit (hereinafter Pₘₐₓ) is defined by the sum of the fluid pressure in the low pressure fluid reservoir (hereinafter P_{L}) and a second pressure (hereinafter P₂) created by a component of the fluid logic system. In other words, Pₘₐₓ = P_{L} + P₂. A person skilled in the art will appreciate that P₁ and P₂ can be different or they can be the same depending on the configuration of the logic system. When the pressure of fluid in the restriction device (hereinafter Pᵣ) is greater than Pₘₐₓ, the fluid logic system will allow fluid flow from the restriction device into the low pressure fluid reservoir. Conversely, when Pᵣ is less than or equal to Pₘₐₓ, the fluid logic system will prevent fluid flow from the restriction device into the low pressure fluid reservoir. A person skilled in the art will appreciate that the logic system can be used to control fluid flow from a high pressure fluid reservoir and/or fluid flow into a low pressure fluid reservoir. Moreover, the pre-set pressure limit can be fixed or adjustable on the high pressure side and/or the low pressure side.

The fluid reservoir(s) 40, 50 used with the fluid logic system 30 can have various configurations, and the system 10 can include any number of reservoirs. For example, the fluid reservoir(s) 40, 50 can be in the form of a housing that is coupled to the fluid logic system 30 by a catheter or other connector, or they can be a housing or chamber formed within the system 30. The fluid reservoir(s) 40, 50 could also be the human body. In an exemplary embodiment, as previously indicated, one of the reservoirs is a high pressure fluid reservoir 40 and the other reservoir is a low pressure fluid reservoir 50. The high pressure fluid reservoir 40 can be, for example, a housing that is effective to contain a fluid under a high pressure. The high pressure can be generated using various techniques know in the art, including various techniques disclosed herein and discussed in more detail below. The low pressure fluid reservoir 50 can likewise be a housing that is effective to contain fluid, but in an exemplary embodiment the low pressure fluid reservoir is the patient's body. A catheter or other pathway can extend from the fluid logic system 30 to a location in the body where it is desirable to release fluid.

FIG. 2A further illustrates an injection port 60 that can optionally be provided to allow fluid or other materials to be introduced into and/or removed from various components of the system, such as the restriction device 20, one or more of the fluid reservoirs 40, 50, or the logic system 30 itself. The injection port 60 can be implanted at a location within the body that is accessible through the tissue. Typically, injection ports are positioned in the lateral subcostal region of the patient's abdomen under the skin and layers of fatty tissue. Surgeons also typically implant injection ports on the sternum of the patient. The particular configuration of the injection port 60 can vary, but typically injection ports include a housing that is configured to be anchored to tissue, and a septum formed in the housing and adapted to receive a delivery device therethrough and to provide access to the system. The illustrated injection port 60 is shown in more detail in FIG. 2B. Again, a person skilled in the art will appreciate that virtually any injection port 60 known in the art can be used with the system, or that the system can be used without any injection port 60.

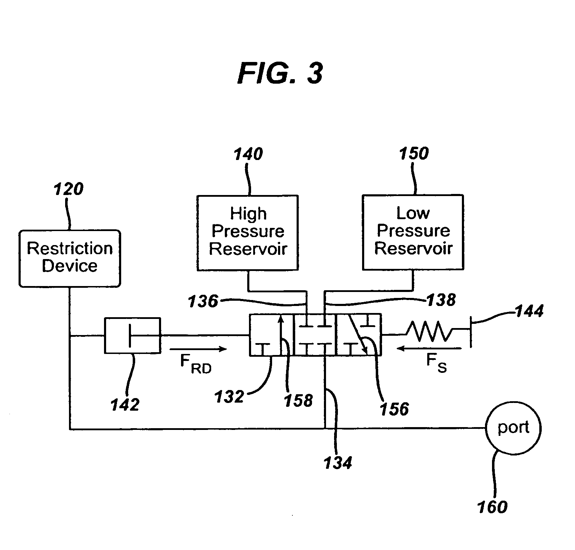

As indicated above, various fluid logic systems known in the art can be used to regulate a pressure of fluid in a restriction device. FIG. 3 illustrates one exemplary fluid logic system 130 that utilizes a fluid logic valve 132. In general, the logic valve 132 includes a first port 134 that is in fluid communication with a restriction device 120, and one or more additional ports that are in communication with one or more fluid reservoirs. In the illustrated embodiment, the logic valve 132 includes a second port 136 that is in fluid communication with a high pressure fluid reservoir 140, and a third port 138 that is in fluid communication with a low pressure fluid reservoir 150. The valve 132 is configured to move in response to changes in pressure of the fluid in the restriction device 120. While the fluid from the restriction device 120 can be in direct communication with the valve 132 to act on the valve 132, FIG. 3 illustrates a transfer mechanism 142 for transferring a force F_{RD} of the fluid from the restriction device 120 to the valve 132. In order to allow the valve 132 to counteract any forces F_{RD} received by the fluid pressure of the restriction device 120 acting thereon, the valve 132 also includes a biasing mechanism 144 that applies a counteracting biasing force F_{S} to the valve 132. The force F_{S} applied to the valve 132 by the biasing mechanism 144 defines the pre-set pressure limits. In particular, the biasing mechanism 144 will define a minimum pre-set pressure limit at which the valve 132 will open to allow fluid to be added to the restriction device 120 from the high pressure fluid reservoir 140, as well as a maximum pre-set pressure limit at which the valve 132 will open to allow fluid to be released from the restriction device 120 into the low pressure fluid reservoir 150. As further shown in FIG. 3, the system can also optionally include an injection port 160 that is in fluid communication with the system 130 to allow fluids to be introduced into and/or removed from the system 130.

In use, in an initial position, as shown, the second and third ports 136, 138 are closed such that fluid flow is prevented between the second and third ports 136, 138 and the first port 134. The restriction device 120 applies the force F_{RD} to the valve 132, and the biasing mechanism 144 applies the counterforce F_{S} to the valve 132 to maintain the valve 132 at a substantial equilibrium. In this position, the force F_{RD} applied to the valve 132 by the fluid in the restriction device 120 is within the pre-set pressure range, i.e., greater than the minimum pre-set pressure limit and less than the maximum pre-set pressure limit. When the pressure in the restriction device 120 changes, the valve 132 will move in response. For example, when the pressure of fluid in the restriction device 120 decreases below the minimum pre-set pressure limit, such as due to patient weight loss, the biasing force F_{S} of the biasing mechanism 144 will be greater than the force F_{RD} applied to the valve 132 by the fluid in the restriction device 120, and thus the valve 132 will move to the left. As a result, the valve 132 will move to a first opened position, in which a fluid pathway 156 is formed between the first port 134 and the second port 136. This will allow fluid to flow from the high pressure fluid reservoir 140 into the restriction device 120, thus increasing a pressure of fluid in the restriction device 120. As the pressure increases, the force F_{RD} applied to the valve 132 by the fluid in the restriction device 120 will overcome the biasing force F_{S} to move the valve 132 back toward the right. When the force F_{RD} applied to the valve 132 by the fluid in the restriction device 120 reaches or exceeds the minimum pre-set pressure limit, the valve 132 will return to the initial position, thus preventing any further fluid flow from the high pressure fluid reservoir 140 into the restriction device 120. Conversely, when the pressure of fluid in the restriction device 120 increases to an amount greater than the maximum pre-set pressure limit, for example when a patient is eating, the force F_{RD} applied to the valve 132 by the fluid in the restriction device 120 will be greater than the biasing force F_{S} applied by the biasing mechanism 144, and thus the valve 132 will move to the right. As a result, the valve 132 will move to a second opened position, in which a fluid pathway 158 is formed between the first port 134 and the third port 138. This will allow fluid to flow from the restriction device 120 into the low pressure fluid reservoir 150, thus decreasing a pressure of fluid in the restriction device 120. While not shown, a fluid pathway can also optionally be formed between the first port 134 and the second port 136 when the valve 132 is in the second opened position. However, such a pathway would preferably include a one-way valve that would allow fluid to flow into the high pressure fluid reservoir 140 and would prevent fluid flow from the high pressure fluid reservoir 140 into the restriction device 120. As fluid is released from the restriction device 120 and the pressure decreases, the biasing force F_{S} applied to the valve 132 by the biasing mechanism 144 will overcome the force F_{RD} applied to the valve 132 by the fluid in the restriction device 120 to move the valve 132 back toward the left. When the force F_{RD} applied to the valve 132 by the fluid in the restriction device 120 is equal to or less than the maximum pre-set pressure limit, the valve 132 will return to the initial position, thus preventing any further fluid flow from the restriction device 120 into the low pressure fluid reservoir 150.

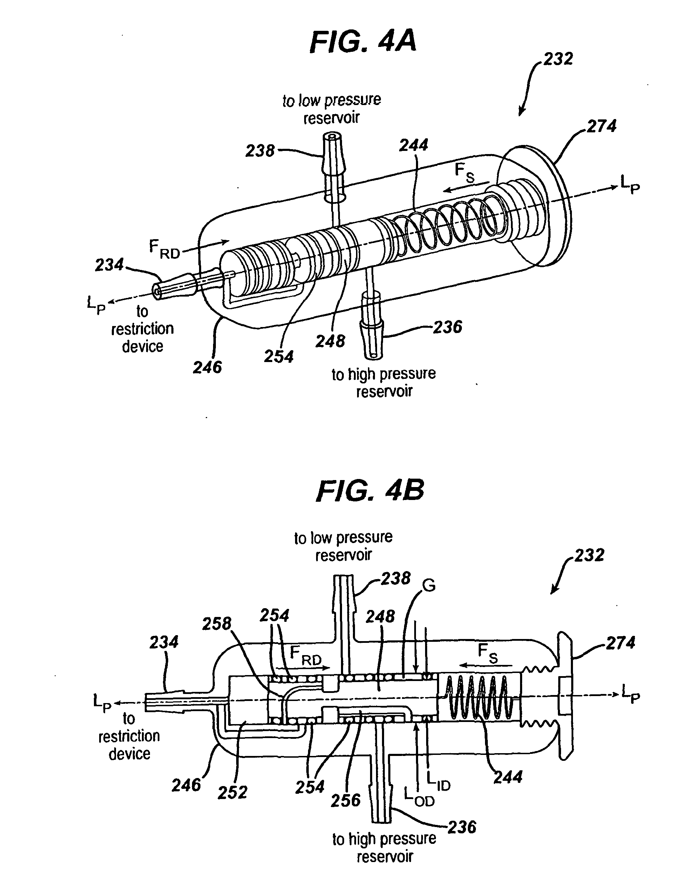

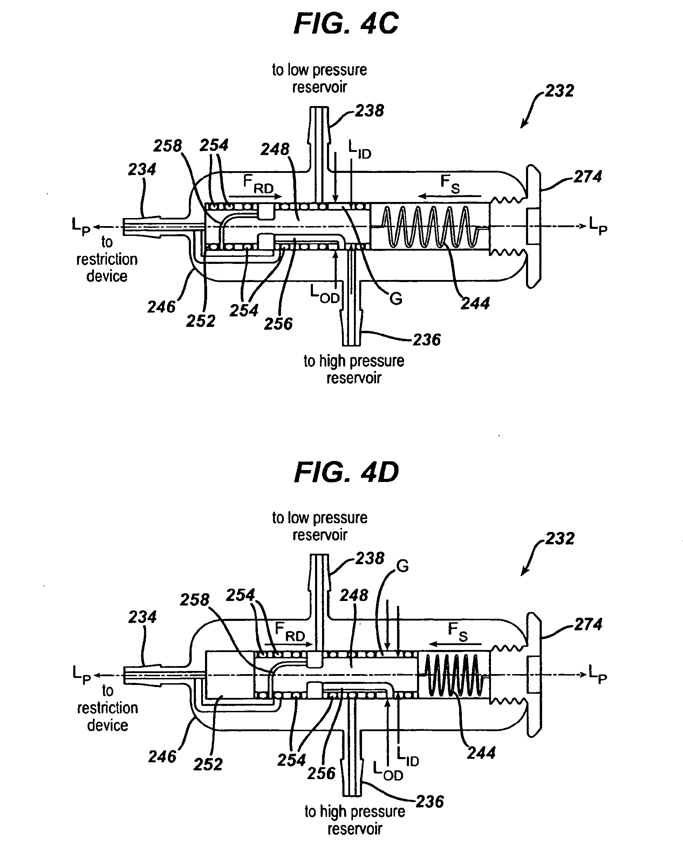

FIGS. 4A-4D illustrate one exemplary embodiment of a logic valve 232. As shown, the valve 232 generally includes a seal and/or piston 248 that is movably disposed within a housing 246. In the illustrated embodiment the piston 248 and housing 246 are cylindrical, however the piston 248 and housing 246 can have various shapes. The housing 246 can include multiple ports formed therein and extending into an inner lumen 252 containing the piston 248. As shown, the housing 246 includes a first port 234 formed in a terminal end thereof, and second and third ports 236, 238 formed in a sidewall thereof. The second and third ports 236, 238 are spaced a distance apart from one another along a longitudinal axis of the housing 246. While the ports can be coupled to various components of the system, in an exemplary embodiment the first port 234 is in fluid communication with a restriction device, the second port 236 is in fluid communication with a high pressure fluid reservoir, and a third port 238 is in fluid communication with a low pressure fluid reservoir. The piston 248, which is disposed within the inner lumen 252, can be configured to control fluid flow between the ports. In an exemplary embodiment, as shown, the piston 248 has an outer diameter L_{OD} that is less than an inner diameter L_{ID} of the inner lumen 252 such that a gap G is formed between the piston 248 and the housing 246. This gap will allow fluid flow therethrough. In order to control the direction of fluid flow, the piston 248 can also include one or more o-rings 254 or similar surface features formed thereon. The o-rings 254 can be spaced along a longitudinal axis L_{P} of the piston 248 and they can be positioned to limit fluid flow from each port to a particular region within the gap G. In order to allow fluid communication between the ports, the piston 248 can also include one or more pathways formed therethrough. In the illustrated embodiment, the piston 248 includes a first pathway 256 that is configured to extend from a region in fluid communication with the second port 236 (high pressure fluid reservoir) to a region in fluid communication with the first port 234 (restriction device), and a second pathway 258 that is configured to extend from a region in fluid communication with the third port 238 (low pressure fluid reservoir) to a region in fluid communication with the first port 234 (restriction device). The first and second pathways 256, 258 can also be configured such that movement of the piston 248 along its longitudinal axis Lp within the lumen will selectively position the pathways 256, 258 in line with the ports. In particular, the piston 248 can have an initial position, as shown in FIG. 4B, in which the first and second pathways 256, 258 are misaligned with the regions in fluid communication with the second and third ports 236, 238. The o-rings 254 will prevent fluid from the second and third ports 236, 238 from flowing into the areas in fluid communication with the pathways 256, 258. The piston 248 can be movable to a first opened position, shown in FIG. 4C, in which the first pathway 256 is aligned with the region in fluid communication with the second port 236 (high pressure fluid reservoir), but the second pathway 258 is misaligned with the region in fluid communication with the third port 238 (low pressure fluid reservoir). Such positioning can allow fluid to flow from the high pressure fluid reservoir, through the second port 236, through the first pathway 256, and out the first port 234 where the fluid is delivered to the restriction device. The o-rings 254 surrounding the third port 238 can prevent fluid from the third port 238 from flowing into the areas in fluid communication with the first and second pathways 256, 258. The piston 248 can also be movable to a second opened position, as shown in FIG. 4D, in which the second pathway 258 is aligned with the region in fluid communication with the third port 238 (low pressure fluid reservoir), but the first pathway 256 is misaligned with the region in fluid communication with the second port 236 (high pressure fluid reservoir). Such positioning can allow fluid from the restriction device to flow into the first port 234, through the second pathway 258, and out of the third port 238 where it is delivered to the low pressure fluid reservoir. The o-rings 254 surrounding the second port 236 can prevent fluid from the second port 236 from flowing into the areas in fluid communication with the first and second pathways 256, 258.

As further shown in FIGS. 4A-4D, the logic valve 232 also includes a biasing mechanism 244 that is coupled to the piston 248 and that is effective to bias the piston 248 in a direction approximately opposite to a force F_{RD} that is applied to the piston 248 by fluid contained within the restriction device. In particular, since the first port 234 is in fluid communication with the restriction device, and the first port 234 is formed in a terminal end of the housing 246, fluid from the restriction device will be in contact with the terminal end of the piston 248 located adjacent to the first port 234. The fluid in the restriction device will thus apply the force F_{RD} to the piston 248. The biasing mechanism 244 can counteract this force with a force F_{S}. The two forces F_{RD}, F_{S} will thus control movement of the piston 248. The biasing mechanism 244 can have a variety of configurations, but in the illustrated embodiment the biasing mechanism 244 is in the form of a coil spring that biases the piston 248 toward the first port 234. As further shown, the biasing mechanism 244 can also be adjustable. For example, the housing 246 can include a screw 274 disposed in a terminal end thereof and coupled to one end of the biasing mechanism 244. Rotation of the screw 274 can be effective to lengthen and/or shorten the biasing mechanism 244, thus increasing and/or decreasing the force F_{S} applied by the biasing mechanisms 244 to the piston 248. In other embodiments, the biasing mechanism 244 could be a fluid-filled bladder, such as a compressible bellows, and the bladder can optionally include a port to allow fluid to be introduced into and removed therefrom to adjust a force that the bladder applies to the piston 248.

In use, the biasing mechanism 244 defines the minimum and maximum pre-set pressure limits. The minimum pre-set pressure limit is the pressure at which, when the pressure of the fluid in the restriction device drops below, the piston 248 moves to the first opened position (i.e., moves to the left) to allow fluid to flow from the high pressure fluid reservoir into the restriction device. The maximum pre-set pressure limit is the pressure at which, when the pressure of the fluid in the restriction device rises above, the piston 248 moves to the second opened position (i.e., moves to the right) to allow fluid to flow from the restriction device into the low pressure fluid reservoir.

A person skilled in the art will appreciate that other components of the logic valve 232 can be altered to define the maximum and minimum pressure limits. For example, the geometry of the piston 248 can be adjusted to change the maximum and minimum pressure limits. In the illustrated embodiment, if the third port 238 were to be positioned further to the right, the maximum and minimum pressure limits would then correspond to different lengths of the biasing mechanism 244.

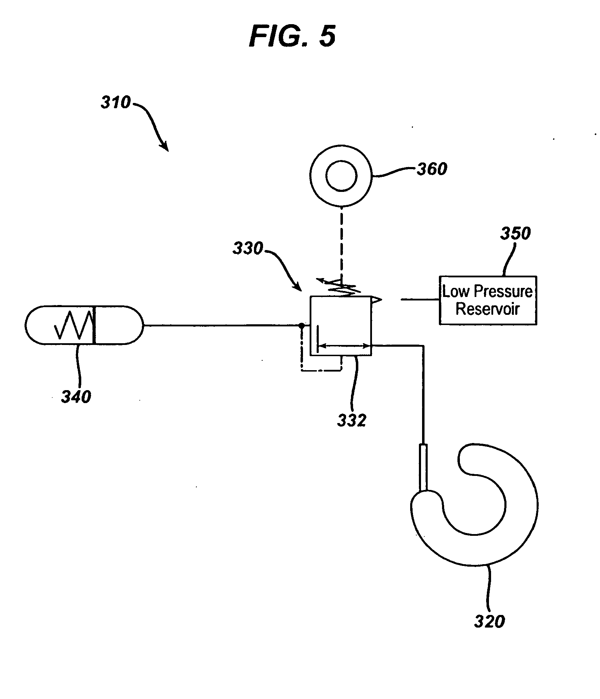

FIG. 5 illustrates another exemplary disclosure of a fluid logic system 330 for use in a restriction system 310. In this embodiment, the fluid logic system 330 utilizes a regulator valve 332. Similar to the valves discussed earlier, the regulator valve 332 is coupled between a restriction device 320 and at least one fluid reservoir. In the illustrated embodiment, there are two fluid reservoirs, a high pressure fluid reservoir 340 and a low pressure fluid reservoir 350, but any number of reservoirs having any pressure can be used. Generally, the regulator valve 332 is configured to move in response to changes in pressure of the fluid in the restriction device 320. Pressures created by components of the regulator valve 332 can operate against the pressure of the fluid in the restriction device 320 such that when the pressures are substantially the same, the regulator valve 332 is closed, and when the pressures are different, the regulator valve 332 is opened. Moreover, the components of the regulator valve 332 can define pre-set pressure limits, such as the pressure at which the regulator valve 332 will open to allow fluid to be added to the restriction device 320 from the high pressure fluid reservoir 340 (a minimum pre-set pressure limit), and the pressure at which the regulator valve will open to allow fluid to be released from the restriction device 320 into the low pressure fluid reservoir 350 (a maximum pre-set pressure limit). The regulator valve 332 can be self-regulating, without the need for adjustments over time as changes occur in the patient, and it can regulate the pressure of the restriction device 320 mechanically without the use of any electrical components that may need to be powered to operate over extended periods of time. The restriction system 310 can also optionally include an injection port 360 that operates similar to the injection port 60 of restriction system 10. As explained with respect to injection port 60, a person skilled in the art will appreciate that virtually any injection port known in the art can be used with the system 310, or that the system 310 can be used without any injection port.

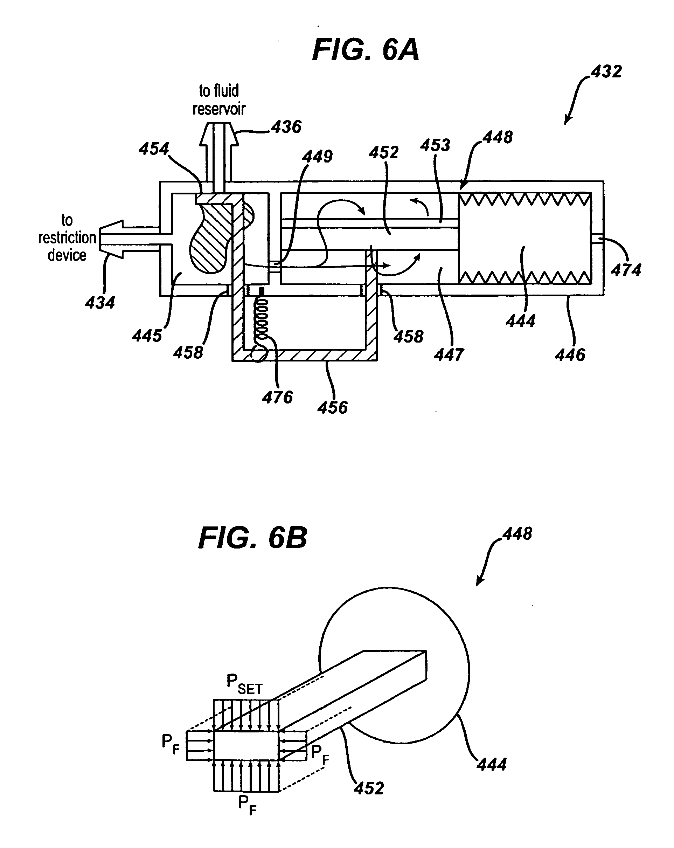

FIGS. 6A and 6B illustrate one exemplary disclosure of a regulator valve 432. The regulator valve 432 generally includes a beam assembly 448 that is movably disposed in a housing 446 having multiple ports formed therein. The housing 446 can have a variety of shapes and sizes, but in the illustrated embodiment the housing 446 is substantially cylindrical. The housing 446 can be configured to contain a fluid that is used in conjunction with the regulator valve 432 and its various components. As shown, a first port 434 is formed in a terminal end of the housing 446 and a second port 436 is formed in a sidewall thereof. While the ports can be coupled to various components of the system, in an exemplary embodiment the first port 434 is in fluid communication with a restriction device and the second port 436 is in fluid communication with a high pressure fluid reservoir. Fluid flow between the ports can be controlled by the beam assembly 448, which can have a variety of configurations. In the illustrated embodiment the beam assembly 448 includes a deflectable beam 452 that is coupled to a seal 454 that is effective to control the flow of fluid through the second port 436 (high pressure fluid reservoir). The deflectable beam 452 is configured to be acted on by a pressure of fluid from the first port 434 (restriction device) and to alter a position of the seal 454 with respect to the second port 436 in response to pressure changes. Movement of the deflectable beam 452 can be translated to the seal 454 by a transfer mechanism, such as the illustrated bar 456. As shown, the bar 456 is mated on one end to the seal 454, extends outside of the housing 446, and is mated on the opposite end to the beam 452. Bushings 458 can optionally be used to assist movement of the transfer mechanism relative to the housing 446. The transfer mechanism can have a variety of different geometries capable of mechanically translating movement of one object to another object, and further, while in the illustrated embodiment the bar 456 extends outside of the housing 446, in other embodiments the bar 456 can be completely disposed within the housing 446.

While the housing 446 can include any number of chambers in the illustrated exemplary embodiment the housing 446 includes two chambers 445, 447 in fluid communication with each other. The first chamber 445 can include the first and second ports 434, 436, the seal 454 for the second port 436, and a portion of the bar 456, and the second chamber 457 can include the deflectable beam 452, another portion of the bar 456, and the biasing mechanism 444. An aperture 449, or other means for transferring fluid from one location to another, such as a pipe, can be located between the first and second chamber 445, 447 to allow the fluid to flow between the first and second chambers 445, 447. This allows fluid from the first port 434 (restriction device) to communicate with the beam, thus allowing the beam to respond to changes in fluid pressure from the first port 434 (restriction device).

In order to allow the deflectable beam 452 to respond to a pressure of fluid from the first port 434 (restriction device), and based on that pressure adjust a position of the seal 454, in one exemplary embodiment the deflectable beam 452 can be bistable. The beam 452 can move between a first position, in which the beam 452 is substantially straight, as shown in FIG. 6A, and a second position, in which the beam 452 buckles. When the beam 452 is in the first position, the seal 454 remains in a closed position to prevent the flow of fluid through the second port 436 (high pressure fluid reservoir) into the first chamber 445 and into the first port 434 (restriction device). However, when the beam 452 is in the second position (not shown), the buckling of the beam 452 allows the seal 454 to open the second port 436 and thus allow fluid to flow from the second port 436 (high pressure fluid reservoir), into the first chamber 445, and into the first port 434 (restriction device). The deflectable beam 452 can be configured in a variety of shapes and sizes, but in an exemplary embodiment the beam 452 includes one or more laminates 453 that are conducive to bistable buckling. The illustrated embodiment shows a single laminate 453 forming a top layer on the deflectable beam 452. Such a configuration facilitates buckling of the beam in a downward direction only. In other embodiments a laminate can be located on a bottom portion of the deflectable beam 452, on both a top and bottom portion of the beam 452, or can be disposed intermittently within and outside of the beam 452. In an exemplary embodiment, the laminate 453 is thin and made of parylene. In one embodiment the laminate is approximately in the range of 0.001 to 0.010 millimeters thick. The beam 452 is capable of buckling because of the various forces acting on the beam 452, which will be described in more detail below.

As indicated above, a biasing mechanism 444 is coupled to the deflectable beam 452 and, in conjunction with the laminate 453, is effective to bias the deflectable beam 452 into a predictable buckled configuration. This biasing force resulting from the biasing mechanism 444 is preferably applied in a direction approximately opposite to a direction of a force that is applied to the beam 452 by fluid from the first port 434 (restriction device). In particular, because the first port 434 is in fluid communication with both the restriction device and the second chamber 447 (via the first chamber 445 and the aperture 449), and the second chamber 447 is where the deflectable beam 452 is located, the fluid in the restriction device surrounds the beam 452 to apply a force to the deflectable beam 452 to bias the beam 452 to the straight configuration shown in FIG. 6A. The biasing mechanism 444 can counteract this force, and together the two forces can control movement of the deflectable beam 452. The forces will be discussed in more detail below with respect to FIG. 6B. The biasing mechanism 444 can have a variety of configurations, but in the illustrated embodiment the biasing mechanism 444 is in the form of a fluid-filled bladder, e.g. a compressible bellows, that biases the deflectable beam 452 into the buckled configuration. Further, the biasing mechanism 444 can also be adjustable. For example, the biasing mechanism 444 can include a port 474 to allow fluid to be introduced into the biasing mechanism 444 and removed therefrom to adjust the force that the biasing mechanism 444 applies to the deflectable beam 452. The system can also optionally include a second biasing mechanism that is effective to help bias the seal 454 to the closed position and the deflectable beam 452 to the straight configuration. As illustrated, the second biasing mechanism is a spring 476 coupled to both the bar 456 and the housing 446. The spring 476 can be adjustable to change the pre-set pressure limit(s).

In use, the biasing mechanism 444 defines the minimum and maximum pre-set pressure limits. As illustrated by FIG. 6B, these pre-set pressure limits, along with other pressures that result from various forces acting on the beam 452, work together to operate the regulator valve 432, and more particularly, deflect and straighten the deflectable beam 452. A pre-set pressure P_{SET} resulting from a force of the biasing mechanism 444 acts on a proximal surface of the beam 452 to apply an approximate downward force on the beam 452. Even though as illustrated the biasing mechanism 444 is coupled to a side of the beam 452 and thus a force of the biasing mechanism 444 acting on the beam can be directed in an approximately horizontal direction, the laminate 453 allows the force being supplied by the biasing mechanism 444 to be directed in an approximately vertical direction. As a result, the biasing mechanism 444 biases the beam 452 to the buckled configuration, which in an exemplary embodiment is in a direction opposite the location of the laminate 453. Meanwhile, a pressure P_{F} resulting from a force of the fluid from the first port 434 (restriction device) acts on the remaining exposed sides of the beam 452, i.e. in the illustrated approximate upward direction and on both sides of the beam 452, thus biasing the beam 452 to the straight configuration.

The minimum pre-set pressure limit is the pressure at which, when the pressure of the fluid in the restriction device drops below, the deflectable beam 452 moves from the first straight position to the second buckled position (i.e., buckles in the approximate downward direction) to allow fluid to flow from the second port 436 (high pressure fluid reservoir) to the first port 434 and into the restriction device to increase a pressure of the fluid in the restriction device. In other words, when the pressure P_{F} applied by the force of the fluid from the restriction device drops below the pressure P_{SET} applied by the force of the biasing mechanism 444, the beam 452 buckles. Buckling movement of the deflectable beam 452 in the approximate downward direction causes the bar 456 and the seal 454 coupled thereto to move in the approximate downward direction, thus opening the port 436 (high pressure fluid reservoir). Fluid flow from the high pressure reservoir into the restriction device will increase a pressure of the fluid in the restriction device. Once the pressure of the fluid of the restriction device is equal to or greater than the minimum pre-set pressure limit, the forces applied to the deflectable beam 452 by the fluid acting thereon from the restriction device will cause the beam 452 to straighten back to the first position. In other words, once the pressure P_{F} applied by the force of the fluid from the restriction device is equal to or greater than the pressure P_{SET} applied by the force of the biasing mechanism 444, the beam 452 will straighten. As a result, the bar 456 will be pulled upward, thus moving the seal 454 to the closed position, thereby preventing the flow of fluid through the second port 436 (high pressure fluid reservoir).

The regulator valve 432 can also have a maximum pre-set pressure limit that can be controlled by way of a valve disposed between a low-pressure fluid reservoir and the restriction device. Although many different types of valves can be used, including the valves disclosed herein, in an exemplary embodiment the valve is a check valve. The check valve can be configured to open to evacuate fluid from the restriction device when the maximum pre-set pressure limit is exceeded. Once the pressure of the fluid of the restriction device is less than or equal to the maximum pre-set pressure, the check valve can be closed.

A person skilled in the art will appreciate that other embodiments of a regulator valve like the regulator valve 432 can include multiple reservoirs, including a low-pressure reservoir, more than two ports, multiple seals, multiple beams, and multiple transfer mechanisms to translate movement of one or more beams to one or more seals. Similarly, in other embodiments a deflectable beam can be adapted to move between more than two positions. For example, a deflectable beam can include a construction that allows it to be adapted to deflect in an approximate upward direction. A person skilled in the art will likewise appreciate that such a design implanting bistable beam elements can be implemented using microelectricalmechanical systems (MEMS) or thin film manufacturing techniques.

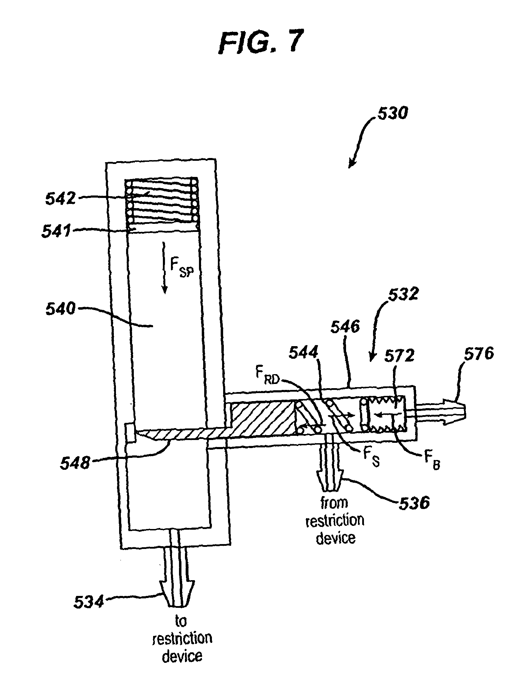

FIG. 7 illustrates another disclosure of a regulator valve 532 for use in a fluid logic system 530. In this embodiment, the fluid logic system 530 generally includes a regulator valve 532 coupled to a high pressure fluid reservoir 540 and configured to regulate a flow of fluid from the fluid reservoir 540 to a restriction device. More particularly, the regulator valve 532 generally includes a chamber 546 and a gate 548 extending between the chamber 546 of the regulator valve 532 and the fluid reservoir 540. Buth the fluid reservoir 540 and the chamber 546 of the regulator valve can have a variety of shapes and sizes, but in the illustrated embodiment both are substantially cylindrical. Neither has to be the same shape or size. As further shown, in one embodiment, the chamber 546 can extend substantially perpendicular to the fluid reservoir 540. Both the fluid reservoir 540 and the chamber 546 can have fluid, such as saline, disposed therein.

Various techniques can be used to form the high pressure fluid reservoir 540, but in one embodiment, as shown, the fluid reservoir 540 can include a spring 542 coupled to a piston 541 such that the spring-piston combination provides a force F_{SP} to pressurize the fluid in the reservoir 540. In one embodiment the spring 542 can be a compression spring. A first port 534 can be located at a terminal end of the fluid reservoir 540 and can be in communication with a restriction device. Fluid flow between the fluid reservoir 540 and the restriction device can be controlled by the gate 548, which as illustrated is at least partially disposed in the fluid reservoir 540.

The gate 548 can have a variety of shapes and sizes that allow it to be configured to move in the fluid reservoir 540 and the chamber 546 of the regulator valve 532 to regulate the flow of fluid from the fluid reservoir 540 to the restriction device. In an exemplary embodiment, the gate 548 is configured to move between an opened position (not shown), in which fluid in the fluid reservoir 540 is free to flow through the first port 534 and into the restriction device, and a closed position (illustrated in FIG. 7), in which the gate 548 prevents fluid in the fluid reservoir 540 from flowing through the first port 534 and into the restriction device. In the illustrated embodiment, a portion of the gate 548 is slidably disposed within the chamber 546 and a second portion of the gate 548 extends into and across the fluid reservoir 540. The gate 548 thus separates the spring 542 and a significant portion of the fluid reservoir 540 from the first port 534, thus acting as a barrier between the high pressure fluid and the first port 534.

In order to allow movement of the gate 548 between the opened and closed positions, a biasing mechanism 544 can be coupled to the gate 548 and can be effective to bias the gate 548 to one of the opened and closed positions. While the biasing mechanism 544 can be a variety of mechanisms capable of biasing the gate 548 toward the opened or closed position, in the illustrated embodiment the biasing mechanism 544 is a tension spring that provides a force F_{S} to biases the gate 548 toward the opened position (to the right as shown). In order to counteract this force, a second port 536 can be located in a sidewall of the chamber 546 and it can be in fluid communication with the restriction device. The second port 536 allows fluid in the restriction device to apply a force F_{RD} to the gate 548 that is operable to counteract the force F_{S}, and thus biases the gate 548 toward the closed position (to the left as shown).

In use, the biasing mechanism 544 defines the minimum pre-set pressure limit. The minimum pre-set pressure limit is the pressure at which, when the pressure of the fluid in the restriction device drops below, the gate 548 moves to the opened position (i.e., moves to the right) to allow fluid to flow from the high pressure fluid reservoir 540, through the first port 534, and into the restriction device. This movement occurs because the pressure exerted by the force F_{S} of the biasing mechanism 544 exceeds the pressure of the force F_{RD} of the fluid of the restriction device, and thus the pressure from the force F_{S} of the biasing mechanism 544 can move the gate 548 toward the opened position to allow fluid to flow into the restriction device to increase the pressure. Once the pressure of the fluid in the restriction device is equal to or greater than then minimum pre-set pressure, the gate 548 can move to the closed position (i.e., moves to the left) to restrict further flow of fluid from the fluid reservoir 540, through the first port 534, and into the restriction device. This movement occurs because the force F_{S} of the biasing mechanism 544 no longer exceeds the force F_{RD} of the fluid of the restriction device, and thus the force F_{RD} of the fluid in the restriction device pushes the gate 548 toward the closed position. In a preferred embodiment, the effect of the pressure from the fluid reservoir 540 on the gate 548 is negligible when compared to the effect of the pressure from the fluid from the restriction device and/or the biasing mechanism 544 on the gate 548.

The regulator valve 532 can also have a maximum pre-set pressure limit that can be controlled by way of a valve disposed between a low-pressure fluid reservoir and the restriction device and/or the chamber 546. Although many different types of valves can be used, including the valves disclosed herein, in an exemplary embodiment the valve is a check valve. The check valve can be configured to open to evacuate fluid from the restriction device when the maximum pre-set pressure limit is exceeded. Once the pressure of the fluid of the restriction device is less than or equal to the maximum pre-set pressure, the check valve can be closed.

As further shown in FIG. 7, an adjustment mechanism 572 can optionally be coupled to the biasing mechanism 544 and it can be configured to change the pre-set pressure limit of the biasing mechanism 544. The adjustment mechanism 572 can have a variety of configurations, but in the illustrated embodiment the adjustment mechanism 572 is in the form of a fluid-filled bladder, e.g. a compressible bellows. The adjustment mechanism 572 can include an adjustment port 576 to allow fluid to be introduced into the adjustment mechanism 572 and removed therefrom to assist in changing the force F_{S} of the biasing mechanism 544. By way of further non-limiting example, another adjustment mechanism that can be used is a screw coupled to the biasing mechanism 544. Rotation of the screw can be effective to increase and/or decrease a force applied by the screw to the biasing mechanism 544, which in turn changes the force F_{S} of the biasing mechanism 544 applied to the gate 548. A person skilled in the art will also appreciate that the biasing mechanism 544 can have particular characteristics that can be adjusted to change the force F_{S} of the biasing mechanism 544. For example, in embodiments where the biasing mechanism 544 is a spring, a spring constant or a length of the spring can be adjusted to change the pre-set pressure limits.

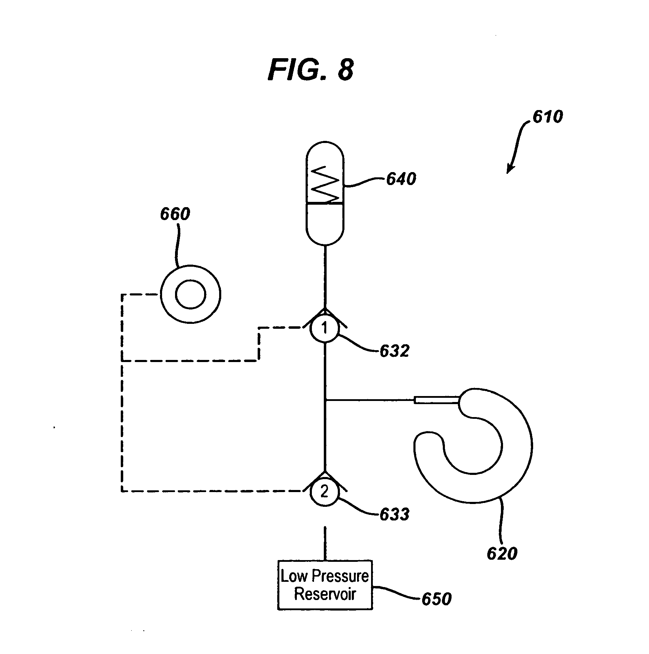

In yet another disclosure the fluid logic system can utilize a check valve. FIG. 8 illustrates one exemplary configuration for a fluid logic system 610 containing a check valve. In this embodiment, the system includes first and second check valves 632, 633, however a person skilled in the art will appreciate that the check valves 632, 633 can be part of the same valve construct, or that they can be separate valve constructs. As shown, the system 610 generally includes the first check valve 632 coupled to a first fluid reservoir 640 and operable to regulate a flow of fluid between the first fluid reservoir 640 and a restriction device 620, and the second check valve 633 coupled to a second fluid reservoir 650 and operable to regulate a flow of fluid between the restriction device 620 and the second fluid reservoir 650. In an exemplary embodiment, the first fluid reservoir 640 can be a high pressure fluid reservoir and the first check valve 632 can be configured to start and stop a flow of fluid from the high pressure fluid reservoir to the restriction device 620 based on one or more designated parameters. Further, the second fluid reservoir 650 can be a low pressure fluid reservoir and the second check valve 633 can be configured to start and stop a flow of fluid from the restriction device 620 to the low pressure fluid reservoir based on one or more designated parameters. In an exemplary embodiment, the first check valve 632 has a minimum pre-set pressure limit and the second check valve 633 has a maximum pre-set pressure limit. The pre-set pressure limits are also referred to as the cracking pressures of the valves. Accordingly, when a pressure of the fluid in the restriction device 620 drops below the minimum pre-set pressure limit, the first check valve 632 moves from a closed position to an opened position to allow fluid to be released from the first fluid reservoir 640 into the restriction device 620 to thereby increase the pressure in the restriction device 620. When the pressure is equal to or greater than the minimum pre-set pressure limit, the first check valve 632 moves from the opened position back to the closed position. Likewise, when the pressure of the fluid in the restriction device 620 exceeds the maximum pre-set pressure limit, the second check valve 633 moves from a closed position to an opened position to allow fluid to be released from the restriction device 620 into the second fluid reservoir 650 to thereby decrease the pressure in the restriction device 620. When the pressure is equal to or less than the maximum pre-set pressure limit, the second check valve 633 moves from the opened position back to the closed position. The check valves 632, 633 can be self-regulating without the need for adjustments over time as changes occur in the patient, and further they can regulate the pressure of the restriction device 620 mechanically without the use of any electrical components that may need to be powered to operate over extended periods of time. The restriction system 610 can also optionally include an injection port 660 that operates similar to the injection port 60 of the restriction system 10. As explained above, a person skilled in the art will appreciate that virtually any injection port known in the art can be used with the system 610, or that the system 610 can be used without any injection port.

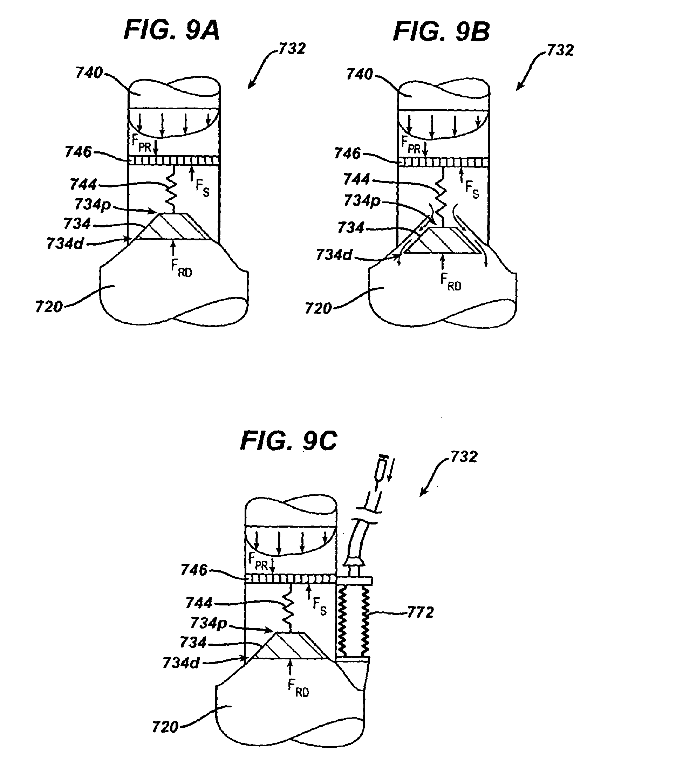

FIGS. 9A and 9B illustrate one exemplary embodiment of a check valve 732. As shown, the check valve 732 includes a plug 734 configured to move between a closed position, illustrated in FIG. 9A in which the plug 734 prevents fluid from flowing between a fluid reservoir 740 and a restriction device 720, and an opened position, illustrated in FIG. 9B in which the plug 734 allows fluid to flow between the fluid reservoir 740 and the restriction device 720. Movement of the plug between the closed and opened positions can result from forces acting on the plug 734 at both a proximal end 734p and a distal end 734d of the plug 734. While the forces acting at either end of the plug 734 can depend on the particular configuration of the check valve and related components, in the illustrated embodiment forces acting on the proximal end 734p can include a force F_{RD} in the approximate downward direction caused by fluid in the fluid reservoir 740 and a force F_{S} in the approximate upward direction caused by a biasing mechanism 744 coupled to the plug 734. As shown, the biasing mechanism 744 is configured to bias the plug 734 in the approximate upward direction, and is thus configured to bias the plug 734 to the closed position. When the pressure in the restriction device 720 changes, the check valve 732 will move in response. For example, when the pressure of fluid in the restriction device 720 decreases below the minimum pre-set pressure limit, such as due to patient weight loss, a biasing force F_{S} of the biasing mechanism 744 (in the approximate upward direction) will be greater than a force F_{RD} applied to the valve 732 by the fluid in the restriction device 720 (in the approximate downward direction), and thus the valve 732 will move toward the opened position (in the approximate downward direction). This will allow fluid to flow from the fluid reservoir 740 into the restriction device 720, thus increasing a pressure of fluid in the restriction device 720. As the pressure increases, the force F_{RD} applied to the valve 732 by the fluid in the restriction device 720 will overcome the biasing force F_{S} to move the valve 732 back toward the closed position (in the illustrated upward direction).

The biasing mechanism 744 can include any number of components configured to bias the plug 734 in a desired direction, but in the illustrated embodiment the biasing mechanism 744 is a spring coupled to the plug 734 at one end of the spring and to a force-receiving plate 746 at a second end of the spring. The force-receiving plate 746 can have various configurations that allow the force created by the fluid from the fluid reservoir 740 to be transferred to the proximal end 734p of the plug 734. Additionally, the biasing mechanism 744 can be removable and/or adjustable to change the amount of force acting on the plug 734. Changing the amount of force exerted by the biasing mechanism 744 on the plug 734 allows a pre-set pressure limit, for example a minimum pre-set pressure and/or a maximum pre-set pressure, to be adjusted for the check valve 732. As the pre-set pressure limits are adjusted, the plug 734 can be set to move to the opened and closed position at different desired pre-set pressures. When the biasing mechanism is a spring, the pre-set pressure limit can be changed by changing the type of spring that is used, which can at least change the spring constant, and/or changing the length of the spring that is used. An exemplary technique for changing the length of the biasing mechanism 744 when the check valve 732 is already implanted is illustrated in FIG. 9C. In this embodiment, the check valve 732 includes a fluid bladder, such as a bellows 772, coupled to the force-receiving plate 746. In use, adding or removing fluid from the bellows 772 changes a height of the force-receiving plate 746, which in turn changes the amount of force applied by the biasing mechanism 744 to the plug 734. Changing the amount of force applied by the biasing mechanism 744 to the plug 734 allows the point at which the plug 734 moves to the opened position or the closed position to be adjusted. Fluid can be added or removed from the bellows 772 in a number of manners to adjust a length of the biasing mechanism 744, and thereby adjust the pre-set pressure limit(s), but preferably it is done non-invasively.

In addition to changing the pre-set pressure limit(s) or cracking pressure(s) of the check valve 732 to affect the point at which the check valve 732 opens and closes, another way to affect the point at which the check valve 732 opens and closes is to change the pressure of the fluid flowing from the reservoir 740 to the check valve 732. For example, while in the illustrated embodiments the pressure of the fluid in the fluid reservoir 740 may decrease as the check valve 732 opens and closes, in another embodiment the fluid reservoir can be a constant pressure reservoir such that the pressure of the fluid in the reservoir remains substantially constant during and after the opening and closing of the check valve 732. Alternatively, the fluid reservoir can be coupled to a constant pressure reservoir such that, even though the fluid reservoir does not maintain a constant pressure on its own, the constant pressure reservoir is capable of maintaining a constant pressure in the fluid reservoir.

A person having ordinary skill in the art will recognize that even though FIGS. 9A-9C are discussed with respect to fluid flowing from the fluid reservoir 740 and into the restriction device 720, the teachings are equally applicable to a system that has a check valve disposed between a fluid reservoir and a restriction device where the check valve is configured to open and close to allow fluid to flow out of the restriction device and into the fluid reservoir.

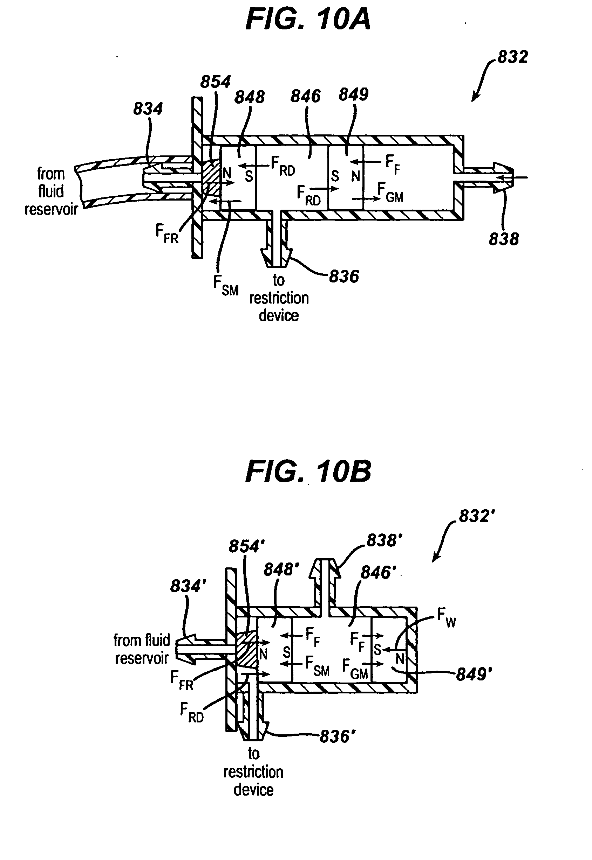

FIGS. 10A and 10B illustrate other exemplary embodiments of a check valve 832, 832'. In the embodiment shown in FIG. 10A, the check valve 832 includes a gasket magnet 848 and a set-point magnet 849 disposed in a chamber 846 and configured to control a flow of fluid between a fluid reservoir and a restriction device. The chamber 846 can have a variety of shapes and sizes, but in the illustrated embodiment the chamber 846 is substantially cylindrical. The chamber 846 can include any number of ports formed therein. As shown, the chamber 846 includes a first port 834 formed in a first end thereof and a second port 836 formed in a sidewall thereof. While the ports can be coupled to various components of the system, in an exemplary embodiment the first port 834 is in fluid communication with a fluid reservoir and the second port 836 is in fluid communication with a restriction device.

The gasket magnet 848 and the set-point magnet 849 can be configured in a number of ways to control the flow of fluid between the first port 834 (fluid reservoir) and the second port 836 (restriction device). As illustrated, the magnets 848, 849 can be disposed in the chamber 846 with opposing poles facing each other, thus repelling each other, and can be adapted to slide therein. In an exemplary embodiment, the gasket magnet 848 is slidable to open and close the first port 834 (fluid reservoir) and thus can have a seal 854 coupled to a left side thereof. The gasket magnet 848 can be effective to move between a closed position, in which the seal 854 occludes the first port 834 to prevent fluid from the fluid reservoir from flowing into the chamber 846 or the restriction device, and an opened position, in which the seal 854 is spaced apart from the first port 834 to allow fluid from the fluid reservoir to flow through the first port 834, into the chamber 846, and then into the second port 836 (restriction device). In alternative embodiments, the gasket magnet 848 itself can serve as the seal. While a location of the gasket magnet 848 can change as a result of sliding between the opened and closed positions, generally the gasket magnet 848 is located between the first and second ports 834, 836. Further, the gasket magnet 848 is preferably sized and shaped to allow fluid to flow between the fluid reservoir and the restriction device. In particular, as shown, the gasket magnet 848 is sized to be smaller than the chamber 846 so that a pathway allows the fluid to travel from the first port 834 (fluid reservoir) to the second port 836 (restriction device). Although in the illustrated embodiment the pathway is disposed below the gasket magnet 848, the pathway can be disposed above or even through the gasket magnet 848.

While the gasket magnet 848 slides between closed and opened positions, the set-point magnet 849 can remain in a fixed position. The fixed position of the set-point magnet 849 can, however, be slidably adjustable to adjust the pre-set pressure limits, as discussed in more detail below. Even though the set-point magnet 849 can be slidably adjustable, the set-point magnet 849 preferably remains spaced apart from the gasket magnet 848 and located to the right of the second port 836. Unlike the gasket magnet 848, the set-point magnet can be sized and shaped to prevent fluid from flowing from one side of the magnet 849 to the other. In particular, as shown, the set-point magnet 849 is sized to generally fit between two sidewalls of the chamber 846. Alternatively, rather than sizing and/or shaping the set-point magnet 849 to prevent the flow of fluid from one side of the set-point magnet 849 to the other, a force-receiving plate configured to both translate a biasing force discussed in more detail below to the set-point magnet 849 and prevent the flow of fluid from one side of the set-point magnet 849 to the other can be coupled to the right side of the set-point magnet 849.

Various forces can act on each of the gasket magnet 848 and the set-point magnet 849 to assist with the control of fluid flow from the first port 834 (fluid reservoir) to the second port 836. In the illustrated embodiment, because the magnets 848, 849 have opposing poles facing each other, a force F_{SM} to the left acts on the gasket magnet 848 and a force F_{GM} to the right acts on the set-point magnet 849. The gasket magnet 848 can also have at least two additional forces acting on it: a force F_{FR} from the fluid of the fluid reservoir and a force F_{RD} from the fluid from the restriction device. The force F_{RD} from the fluid of the restriction device can also act on the set-point magnet 849.

In use, the set-point magnet 849 defines the minimum pre-set pressure limit. The minimum pre-set pressure limit is the pressure at which, when the pressure of the fluid in the restriction device drops below, the gasket magnet 848 moves to the opened position (i.e., moves to the right) to allow fluid to flow from the first port 834 (fluid reservoir), through the second port 836, and into the restriction device. This movement occurs because the pressure exerted by the force F_{FR} of the fluid reservoir exceeds the pressure of the combined forces F_{RD} of the fluid of the restriction device and F_{SM} of the set-point magnet 849, and thus the pressure from the force F_{FR} of the fluid reservoir can move the gasket magnet 848 toward the opened position to allow the pressure of the fluid of the restriction device to increase. Once the pressure of the fluid of the restriction device is equal to or greater than the minimum pre-set pressure, the gasket magnet 848 can move to the closed position (i.e., moves to the left) to restrict further flow of fluid from the first port 834 (fluid reservoir), through the second port 836, and into the restriction device. This movement occurs because the pressure resulting from the force F_{FR} of the fluid reservoir no longer exceeds the pressure of the combine forces F_{RD} of the fluid of the restriction device and F_{SM} of the set-point magnet 849, and thus the pressure from the combined forces F_{RD} of the fluid of the restriction device and F_{SM} of the set-point magnet 849 push the gasket magnet 848 toward the closed position.

The check valve 832 can also have a maximum pre-set pressure limit that can be controlled by way of a second valve disposed between a low-pressure fluid reservoir and the restriction device and/or the chamber 846. Although many different types of valves can be used, including the valves disclosed herein, in an exemplary embodiment the second valve is a second check valve. The second check valve can be configured to open to evacuate fluid from the restriction device when the maximum pre-set pressure limit is exceeded. Once the pressure of the fluid of the restriction device is less than or equal to the maximum pre-set pressure, the second check valve can be closed.

In another embodiment, the force F_{SM} of the set-point magnet 849 can be adjustable. Adjusting the force F_{SM} of the set-point magnet 849 can likewise adjust the pre-set pressure limit because of the effect of the force F_{SM} of the set-point magnet 849 on the gasket magnet 848. Generally, the closer the set-point magnet 849 is to the gasket magnet 848, the greater the force F_{SM} is that acts on the gasket magnet 848. One way to change the amount of force acting on the gasket magnet 848 is to change various properties of either or both of the gasket and set-point magnets 848, 849 themselves by, for example, using a different material or adjusting the size. In the illustrated embodiment, the force F_{SM} of the set-point magnet 849 can be adjusted by a biasing mechanism. As shown, the biasing mechanism is a fluid disposed to the right of the set-point magnet 849. The chamber 846 can include a third port 838 formed in a second end of the chamber 846 and configured to receive a fluid. The fluid can generally be incompressible. Adjustment of an amount or type of fluid in the chamber 846 to the right of the set-point magnet 849 can subsequently adjust a position of the set-point magnet 849. For example, in the illustrated embodiment, adding fluid via the third port 838 increases a force F_{F} applied to the set-point magnet 849 acting to the left as illustrated, which in turn causes the set-point magnet 849 to move closer to the gasket magnet 848. Moving the set-point magnet 849 close to the gasket magnet 848 subsequently increases the force F_{SM} acting on the gasket magnet 848. Further, biasing mechanisms such as expandable bladders, springs, and screws, as discussed with respect to other embodiments, can also be incorporated into the design of the check valve 832 to adjust the pre-set pressure of the system. Likewise, similar to the other described check valve system, the pressure of the fluid in the fluid reservoir can also be adjusted, or made constant, to adjust the pre-set pressure of the check valve 832.

In another embodiment of a check valve 832', shown in FIG. 10B, the valve 832' is similar to the check valve 832 shown in FIG. 10A except that the location of the set-point magnet 849' is fixed in the chamber 846'. Accordingly, a third port 838', which is configured to adjust a pre-set pressure of the check valve 832', is disposed in a sidewall of the chamber 846' between the gasket magnet 848' and the set-point magnet 849'. The embodiment illustrated in FIG. 10B is also different than the embodiment illustrated in FIG. 10A because the gasket magnet 848' is not generally sized and shaped to allow fluid to flow from one side of the gasket magnet 848' to the other. This is because, as illustrated, there is no space between the chamber 846' and the gasket magnet 848' or in the gasket magnet 848' itself. Accordingly, the second port 836' is disposed to the left of the gasket magnet 848' so that when the gasket magnet 848', which can include a seal 854', moves to an opened position, fluid can flow from the first port 834' (fluid reservoir) to the second port 836' (restriction device).

The forces created by the parameters related to the set-point magnet 849' can be adjusted in many of the same ways as discussed with respect to the set-point magnet 849 of FIG. 10A. Because this illustrated embodiment is a bit different though, while adding fluid to the system through the third port 838' does adjust the pre-set pressure, it does so without moving the set-point magnet 849'. Rather, to adjust the pre-set pressure, in one embodiment a desired amount of diamagnetic or paramagnetic fluid can be introduced or removed from the chamber 846' between the gasket and set-point magnets 848', 849' to respectively increase or decrease the effective magnetic force between the two magnets 848', 849', which in turn adjusts a force F_{SM} acting on the gasket magnet 848' in the illustrated approximate left direction. Further, because this embodiment is a bit different than the embodiment illustrated in FIG. 10A, the forces acting on the gasket magnet 848' and the set-point magnet 849' are also a bit different. As illustrated, the forces acting on the gasket magnet 848' in an approximate left direction include a force F_{FR} caused by fluid from the fluid reservoir and a force F_{RD} caused by fluid from the restriction device, and the forces acting in an approximate right direction include the force F_{SM} resulting from the set-point magnet and a force F_{F} caused by the fluid introduced through the third port 838'. As further illustrated, the forces acting on the set-point magnet 849' in an approximate right direction include a force F_{GM} resulting from the gasket magnet and a force F_{F} caused by the fluid introduced through the third port 838', and a force acting in an approximate left direction includes a force F_{w} of a wall of the check valve 832'.

A person having ordinary skill in the art will recognize that even though FIGS. 10A and 10B are discussed with respect to a fluid flowing from the first port 834, 834' (fluid reservoir), through the second port 836, 836', and into the restriction device, the teachings are equally applicable to a system that has a check valve disposed between a fluid reservoir and a restriction device where the check valve is configured to open and close to allow fluid to flow out of the restriction device and into the fluid reservoir. Further, a person skilled in the art will also recognize that, although directed to a magnetic check valve, the teachings can also be applied to the fluid reservoir to regulate the flow of fluid from the reservoir to the check valve, or alternatively, in instances where multiple fluid reservoirs are coupled together, between the multiple fluid reservoirs.

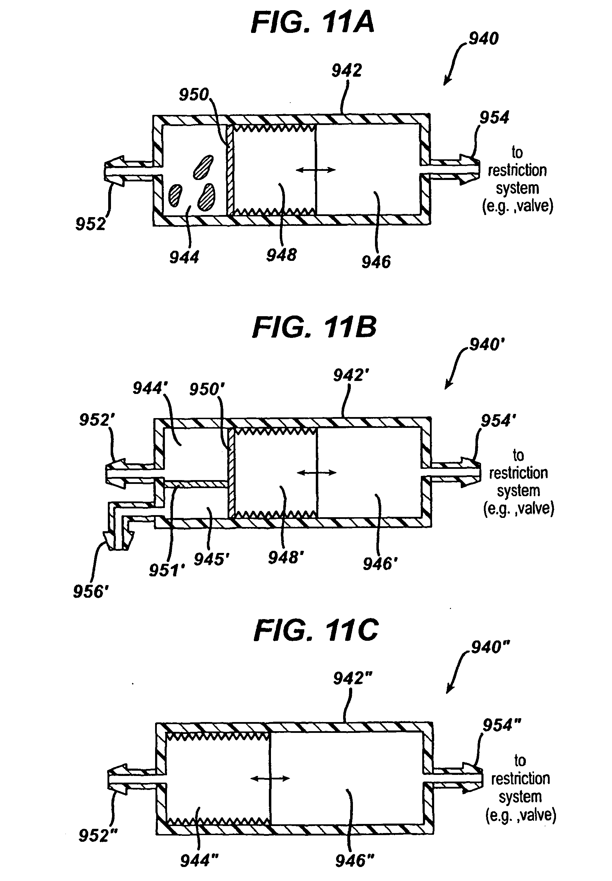

As previously indicated, the various fluid logic systems disclosed herein can include a high pressure fluid reservoir and various techniques can be used to generate a high pressure within a fluid reservoir. In fact, as discussed with respect to the magnetic check valves, many of the embodiments and techniques discussed with respect to adjusting and regulating the flow of check valves can also be applied to adjusting and controlling the flow of fluid into and/or out of one or more fluid reservoirs. By way of further non-limiting example, FIGS. 11A-11C illustrate various embodiments of high pressure fluid reservoirs. In particular, the illustrated high pressure fluid reservoirs are configured to release and absorb heat, gas, or other products resulting from one or more chemical reactions to control an amount of fluid flow from the high pressure fluid reservoirs to other components of a restriction system, such as valves, restriction devices, and fluid reservoirs.

In the illustrated embodiment of FIG. 11A, the high pressure fluid reservoir 940 generally includes a housing 942 with multiple ports formed therein, a chemical reaction chamber 944, a fluid chamber containing fluid adapted for use in a restriction device, such as saline, and an expandable bladder 948 disposed between the two chambers 944, 946. In one embodiment the expandable bladder 948 is filled with a substance having a defined volume-temperature function, such as air. Optionally, a transfer element 950 can be coupled to the expandable bladder. The transfer element 950 can be configured to transfer forces, temperatures, or other parameters from the chemical reaction chamber 944 to the expandable bladder 948.

The housing 942 can have a variety of shapes and sizes, but in the illustrated embodiment the housing 942 is substantially cylindrical. As shown, a first port 952 is formed in a first end of the housing 942 and a second port 954 is formed in a second end thereof. While the ports can be coupled to various components of the system, in an exemplary embodiment the first port 952 is configured to receive one or more chemicals for use in chemical reactions and the second port 954 is in fluid communication with a valve or other component of a restriction system. In some embodiments, depending on the chemical reaction(s) used, the first port can be excluded, for instance if access to the chemical reaction chamber 944 is not needed for the operation of the high pressure fluid reservoir 940. Fluid flow from the fluid chamber 946 to the valve is controlled, at least in part, by chemical reaction(s) performed in the chemical reaction chamber 944. At least a portion of the resulting product of the chemical reaction(s) can adjust a force applied by the expandable bladder 948 to the fluid chamber 946, which in turn adjusts an amount of fluid flowing from the fluid chamber 946 to the valve or other component of a restriction system. In a preferred embodiment, the chemical reaction(s) are reversible, and thus the same fluid chamber 946 can be used to both increase and decrease the rate and/or amount of fluid flowing from the fluid chamber 946 to the valve or other component of a restriction system.

Chemical reaction(s) are generally initiated in one of two ways. In one method, one or more chemicals disposed in the chemical reaction chamber 944 can be selected such that the reaction is not instantaneous and thus the results of the reaction can occur at some point after the introduction of the chemicals into the chemical reaction chamber 944. In another method, one or more chemicals can be added to the chemical reaction chamber 944 through the first port 952 to cause a desired chemical reaction. In an exemplary embodiment, all of the chemicals needed to generate the desired chemical reaction are disposed in the chemical reaction chamber 944 except one, and the last chemical is added via the first port 952 to begin the desired chemical reaction. A person skilled in the art will recognize that any number of chemicals can be used in the chemical reaction chamber 944 and any number can be added to the chemical reaction chamber 944, depending at least in part on the chemical reaction involved and the desired result. The desired chemical reaction can be effective to move the expandable bladder 948, which in turn affects the flow of fluid from the fluid chamber 946 to the valve or other components of a restriction system. For example, the resulting reaction can be an exothermic reaction, which releases heat thus causing the expandable bladder 948 to expand and apply additional force to the fluid in the fluid chamber 946. This results in an increase in the amount of fluid flowing out of the fluid chamber 946, which can also increase the amount of force and pressure being applied to the valve or other component of a restriction system, depending on the other components of the system. An example of an exothermic reaction that can work in such an embodiment includes mixing water with strong acids. Similarly, an endothermic reaction, which absorbs heat, can also be used. An endothermic reaction can cause the expandable bladder 948 to contract and thus apply less force to the fluid in the fluid chamber 946 than before the endothermic reaction, which in turn results in a decrease of the amount of fluid flowing out of the fluid chamber 946, or alternatively causes fluid to flow into the chamber 946. An endothermic reaction can also decrease the amount of force and pressure being applied to the valve or other component of a restriction system coupled to the second port 954. Chemical reactions are not limited to just exothermic and endothermic reactions however. Many other types of reactions can also be used to increase or decrease an amount of force applied to fluid disposed in the fluid chamber 946. Often times these reactions can produce by-products, such as gas. The release of gas and other products can cause the pressure within the chemical reaction chamber 944 to increase, which in turn increases a force applied to the expandable bladder 948. One example of a gas releasing reaction that can be used is NaHCO₃ + HCL → NaCl + H₂0 +CO₂. A second example of a gas releasing reaction that can be used is a combination of XCO₃ and an acid.

The embodiment shown in FIG. 11B is similar to FIG. 11A except that it includes multiple chemical reaction chambers and more than two ports. As shown, the housing 942' can include first, second, and third ports 952', 954', and 956', respectively, formed therein. Further, an expandable bladder 948' can be disposed in the housing 942' and can separate a fluid chamber 946' from first and second chemical reaction chambers 944', 945'. In one embodiment the expandable bladder 948' is filled with a substance having a defined volume-temperature function, such as air. Further, a transfer element 950' can optionally be coupled to the expandable bladder 948' and configured to transfer forces, temperatures, or other parameters from the chemical reaction chambers 944', 945' to the expandable bladder 948'.

Each of the ports 952', 954', 956' can be coupled to various components of the system, but in the illustrated embodiment the second port 954' is in fluid communication with a valve or other component of a restriction system such that fluid from the fluid chamber 946' can move between the fluid chamber 946' and the valve or other components of the restriction system. Further, the first port 952' is configured to receive one or more chemicals for use in chemical reactions in the first chemical reaction chamber 944' and the third port 956' is configured to receive one or more chemicals for use in chemical reactions in the second chemical reaction chamber 945'. Optionally, a gate 951' can be disposed between the first and second chemical reaction chambers 944', 945' to allow for communication between the chambers 944', 945' and/or the ports 952', 956'. In some embodiments, depending on the chemical reaction(s) used, the first and third ports 952', 956' can be excluded, for instance if access to the chemical reaction chambers 944', 945' is not needed for the operation of the high pressure fluid reservoir 940'. Further, in an exemplary embodiment, each chemical reaction chamber 944'; 945' can be configured for a different type of reaction. For example, in instances where an exothermic reaction is used to expand the expandable bladder 948' and an endothermic reaction is used to contract the expandable bladder 948', the first chemical reaction chamber 944' can be configured for exothermic reactions and the second chemical reaction chamber 945' can be configured for endothermic reactions. This embodiment is advantageous because non-reversible chemical reactions can be easily used in the chemical reaction chambers 944', 945'. Use of the high pressure fluid reservoir 940' is similar to use of the high pressure fluid reservoir 940 illustrated in FIG. 11A, and thus the same principles can be applied to this embodiment.

FIG. 11C illustrates yet another embodiment of a high pressure fluid reservoir 940" that is similar to the embodiment illustrated in FIG. 11A, however the chemical reaction chamber 944" is an expandable bladder. As shown, a first port 952" is formed on a proximal end of the housing 942" and provides access to the chemical reaction chamber 944", i.e. the expandable bladder, and a second port 954" is formed on a terminal end of the housing 942" and is in fluid communication with a valve or other component of a restriction system. Similar to the high pressure fluid reservoir 940 of FIG. 11A, the chemical reaction(s) can generally be initiated in one of two ways, with the first method not requiring the addition of any further chemicals via the first port 952", as described earlier, and the second method requiring the addition of at least one chemical through the first port 952 to generate a desired chemical reaction. Likewise, use of the high pressure fluid reservoir 940" is similar to use of the high pressure fluid reservoir 940 discussed with respect to FIG. 11A, and thus the same principles can be applied to this embodiment. By way of non-limiting example, in one exemplary embodiment a chemical is added to the chemical reaction chamber 944" that results in a gas releasing reaction. The release of gas causes the chemical reaction chamber 944 to expand, which in turn increases the amount of the flow of fluid flowing out of the fluid chamber 946". This can also increase the amount of force and pressure being applied to the valve or other components of the restriction system, depending on the other components of the system coupled thereto.

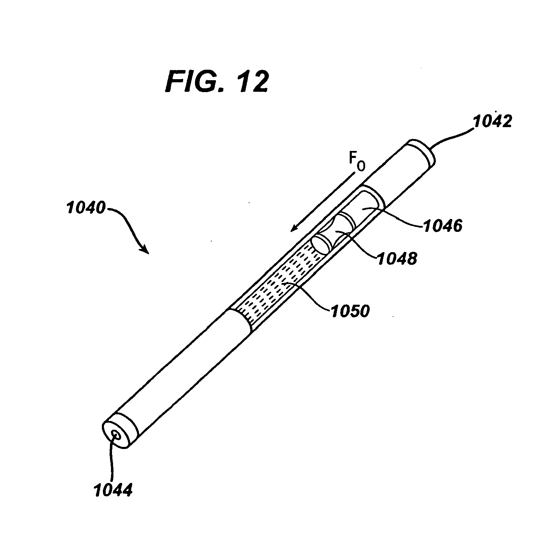

FIG. 12 illustrates yet another non-limiting example of a high pressure fluid reservoir for use in a fluid logic system, such as the systems disclosed herein. As shown, an osmotic pump 1040 is provided and generally includes a housing 1042 with a first end having a semi-permeable membrane 1044 and a second end having an exit port 1044. The housing 1042 can include an osmotic chamber 1046 (sometimes referred to as an osmotic engine) having an osmotic substance, such as a salt-like solution, contained therein. The housing 1042 can also include a piston 1048 and a fluid 1050 disposed therein. In the illustrated embodiment, the osmotic chamber 1046 is located adjacent to the semi-permeable membrane 1044, the fluid 1050 is located adjacent to the exit port 1044, and the piston 1048 is disposed between the osmotic chamber 1046 and the fluid 1050. In use, an osmotic fluid can permeate through the semi-permeable membrane to cause a reaction in the osmotic chamber 1046, thereby actuating the piston 1048 to push the fluid 1050 from the housing 1042 out of the osmotic pump 1040 through the exit port 1044. For example, the osmotic fluid can enter the osmotic chamber 1046 and can expand the salt-like solution contained therein. Expansion of the salt-like solution in the osmotic chamber 1046 can cause a force F_{O} to be applied on the piston 1048, which in turn can cause the piston to move toward the exit port 1044 in the direction of the force F_{O}. Such movement of the piston 1048 can subsequently cause the fluid 1050 to push toward the exit port 1044 and exit the osmotic pump 1040 as a high pressure fluid. The amount and rate of the fluid 1050 flowing through the exit port 1044 can be regulated using various techniques known in the art.

A person skilled in the art will recognize that although the teachings related to the embodiments in FIGS. 11A-11C and FIG. 12 are directed to use in a high pressure fluid reservoir, the teachings can also be used in devices that regulate the flow of fluid from a fluid reservoir to a restriction device, such as a valve, or alternatively, can be used directly with restriction devices to expand or contract the restriction device. Moreover, various other techniques known in the art for creating a high pressure fluid reservoir can be used.

To the extent that any of the fluid logic systems, high pressure fluid reservoirs, other devices and systems, and/or components thereof incorporate springs or other mechanical components that can be adjusted to provide different dimensions or properties (such as spring constants), a person skilled in the art will appreciate that changes to many of the properties and dimensions will affect the performance of the respective fluid logic systems, high pressure fluid reservoirs, other devices and systems, and/or components thereof. Accordingly, even if changes to these types of components are not discussed above, such changes could be incorporated into many of the fluid logic systems, high pressure fluid reservoirs, other devices and systems, and/or components thereof to affect the desired performance of each.

A person skilled in the art will appreciate that the present invention has application in conventional endoscopic and open surgical instrumentation as well application in robotic-assisted surgery.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred that device is sterilized. This can be done by any number of ways known to those skilled in the art including beta or gamma radiation, ethylene oxide, steam.

One skilled in the art will appreciate further features and advantages of the invention based on the above-described embodiments. Accordingly, the invention is not to be limited by what has been particularly shown and described, except as indicated by the appended claims.

## Claims

1. A self-regulating restriction system, comprising:
a restriction device (120) configured to receive fluid and to form a restriction in a pathway corresponding to an amount of fluid contained therein; and
a fluid logic system (130) coupled to the restriction device and configured to regulate an amount of fluid in the restriction device in response to a fluid pressure acting thereon, wherein the fluid logic system includes at least one fluid reservoir (140, 150) and at least one valve coupled between the at least one fluid reservoir and the restriction device;
**characterised in that** the at least one valve comprises a logic valve (132) wherein the logic valve is configured to regulate fluid flow in response to a pressure of fluid in the restriction device acting thereon;
further comprising a biasing mechanism (144) coupled to the logic valve and effective to apply a biasing force to the logic valve that acts against a force applied to the logic valve by a pressure of fluid in the restriction device.

2. The system of claim 1, wherein the fluid logic system defines at least one pre-set pressure limit, and when a pressure of fluid in the restriction device is less than or greater than the at least one pre-set pressure limit the at least one valve moves from a closed to an opened position to allow fluid to flow between the at least one fluid reservoir and the restriction device.

3. The system of claim 2, wherein the at least one fluid pressure fluid reservoir comprises a high pressure fluid reservoir (140) in fluid communication with the restriction device, and the at least one pre-set pressure limit comprises a minimum pre-set pressure limit, and wherein when the pressure of fluid in the restriction device is less than the minimum pre-set pressure limit the at least one valve is configured to open to allow fluid flow from the high pressure fluid reservoir into the restriction device.

4. The system of claim 2, wherein the at least one fluid pressure fluid reservoir comprises a low pressure fluid reservoir (150) in fluid communication with the restriction device, and the at least one pre-set pressure limit comprises a maximum pre-set pressure limit, and wherein when the pressure of fluid in the restriction device is greater than the maximum pre-set pressure limit the at least one valve is configured to open to allow fluid flow from the restriction device into the low pressure fluid reservoir.

5. The system of claim 2, wherein the pre-set pressure limit includes a maximum pre-set pressure limit and a minimum pre-set pressure limit, and wherein the at least one valve is configured to open to release fluid from the at least one reservoir into the restriction device when the pressure of fluid in the restriction device is less than the minimum pre-set pressure limit, and the at least one valve is configured to open to release fluid from the restriction device into the at least one reservoir when the pressure of fluid in the restriction device is greater than the maximum pre-set pressure limit.

6. The system of claim 5, wherein the at least one fluid pressure fluid reservoir comprises a high pressure fluid reservoir in fluid communication with the restriction device and a low pressure fluid reservoir in fluid communication with the restriction device, and the at least one valve is configured to release fluid from the high pressure fluid reservoir and into the restriction device and to release fluid from the restriction device into the low pressure fluid reservoir.

7. The system of claim 2, wherein the at least one pre-set pressure limit is adjustable.

8. The system of claim 7, wherein the at least one pre-set pressure limit is adjustable by adjusting a pressure of fluid in the at least one fluid reservoir.

9. The system of claim 7, wherein the at least one pre-set pressure limit is adjustable by adjusting a parameter of the at least one valve.

10. The system of claim 1, wherein the biasing mechanism is adjustable to allow the biasing force to be adjusted.

11. The system of claim 1, wherein the logic valve includes a first port (134) in fluid communication with the at least one fluid reservoir, a second port (136) in fluid communication with the restriction device, and at least one seal configured to regulate fluid flow between the first and second ports to thereby regulate fluid flow between the at least one fluid reservoir and the restriction device acting thereon.

## Patentansprüche

1. Selbstregulierendes Restriktionssystem, das aufweist:
eine Restriktionseinheit (120), die so ausgelegt ist, dass sie Fluid aufnimmt und dass sie eine Verengung in einem Durchgang bildet, entsprechend einer Menge an Fluid, das darin enthalten ist; und
ein Fluidlogiksystem (130), das mit der Restriktionseinheit gekoppelt ist und so ausgelegt ist, dass es eine Menge an Fluid in der Restriktionseinheit reguliert, als Antwort auf einen Fluiddruck, der darauf wirkt, wobei das Fluidlogiksystem wenigstens einen Fluidbehälter (140, 150) und wenigstens ein Ventil, das zwischen den wenigstens einen Fluidbehälter und die Restriktionseinheit gekoppelt ist, umfasst;
**dadurch gekennzeichnet, dass** das wenigstens eine Ventil ein Logikventil (132) aufweist, wobei das Logikventil so ausgelegt ist, dass es den Fluidstrom als Antwort auf einen Druck des Fluides in der Restriktionseinheit, der darauf wirkt, reguliert;
weiter mit einem vorbelastenden Mechanismus (144), der an das Logikventil gekoppelt ist und so wirkt, dass er eine vorbelastende Kraft auf das Logikventil ausübt, die gegen eine Kraft wirkt, die auf das Logikventil durch einen Druck des Fluides in der Restriktionseinheit ausgeübt wird.

2. System nach Anspruch 1, bei dem das Fluidlogiksystem wenigstens eine vorab eingestellte Druckgrenze definiert, und wenn ein Druck des Fluides in der Restriktionseinheit kleiner oder größer als die wenigstens eine vorab eingestellte Druckgrenze ist, sich das wenigstens eine Ventil aus einer geschlossenen in eine offene Position bewegt, um zu ermöglichen, dass Fluid zwischen dem wenigstens einen Fluidbehälter und der Registriktionseinheit strömt.

3. System nach Anspruch 2, bei dem der wenigstens eine unter Fluiddruck stehende Fluidbehälter einen unter hohem Druck stehenden Fluidbehälter (140) in fluidischer Verbindung mit der Restriktionseinheit aufweist und die wenigstens eine vorab eingestellte Druckgrenze einen minimalen vorab eingestellten Druckgrenzwert aufweist und wobei, wenn der Druck des Fluides in der Restriktionseinheit kleiner ist als der minimale vorab eingestellte Druckgrenzwert, das wenigstens eine Ventil so ausgelegt ist, dass es sich öffnet, um zu ermöglichen, dass Fluid aus dem unter Hochdruck stehenden Fluidbehälter in die Restriktionseinheit strömt.

4. System nach Anspruch 2, bei dem der wenigstens eine unter Fluiddruck stehende Fluidbehälter einen unter niedrigem Druck stehenden Fluidbehälter (150) in fluidischer Verbindung mit der Restriktionseinheit aufweist und die wenigstens eine vorab eingestellte Druckgrenze einen maximalen vorab eingestellten Druckgrenzwert aufweist und wobei, wenn der Druck des Fluides in der Restriktionseinheit größer ist als der maximale vorab eingestellte Druckgrenzwert, das wenigstens eine Ventil so ausgelegt ist, dass es sich öffnet, um zu ermöglichen, dass Fluid aus der Restriktionseinheit in den unter niedrigem Druck stehenden Fluidbehälter strömt.

5. System nach Anspruch 2, bei dem die vorab eingestellte Druckgrenze einen maximalen vorab eingestellten Druckgrenzwert und einen minimalen vorab eingestellten Druckgrenzwert umfasst und bei dem das wenigstens eine Ventil so ausgelegt ist, dass es sich öffnet, um Fluid aus dem wenigstens einen Behälter in die Restriktionseinheit zu entlassen, wenn der Druck des Fluides in der Restriktionseinheit kleiner ist als der minimale vorab eingestellte Druckgrenzwert, und das wenigstens eine Ventil so ausgelegt ist, dass es sich öffnet, um Fluid aus der Restriktionseinheit in den wenigstens einen Behälter zu entlassen, wenn der Druck des Fluides in der Restriktionseinheit größer ist als der maximale vorab eingestellte Druckgrenzwert.

6. System nach Anspruch 5, bei dem der wenigstens eine unter Fluiddruck stehende Fluidbehälter einen unter hohem Druck stehenden Fluidbehälter in fluidischer Verbindung mit der Restriktionseinheit und einem unter niedrigem Druck stehenden Fluidbehälter in fluidischer Verbindung mit der Restriktionseinheit aufweist und das wenigstens eine Ventil so ausgelegt ist, dass es Fluid aus dem unter hohem Druck stehenden Fluidbehälter und in die Restriktionseinheit auslässt und Fluid aus der Restriktionseinheit in den unter niedrigem Druck stehenden Fluidbehälter auslässt.

7. System nach Anspruch 2, bei dem die wenigstens vorab eingestellte Druckgrenze anpassbar ist.

8. System nach Anspruch 7, bei dem die wenigstens eine vorab eingestellte Druckgrenze anpassbar ist, indem ein Druck des Fluides in dem wenigstens einen Fluidbehälter angepasst wird.

9. System nach Anspruch 7, bei dem die wenigstens eine vorab eingestellte Grenze anpassbar ist, indem ein Parameter des wenigstens einen Ventils angepasst wird.

10. System nach Anspruch 1, bei dem der vorbelastende Mechanismus anpassbar ist, um zu ermöglichen, dass die vorbelastende Kraft angepasst werden kann.

11. System nach Anspruch 1, bei dem das Logikventil einen ersten Port (134) in fluidischer Verbindung mit dem wenigstens einen Fluidbehälter, einen zweiten Port (136) in fluidischer Verbindung mit der Restriktionseinheit und wenigstens eine Dichtung umfasst, die so ausgelegt ist, dass sie den Fluidstrom zwischen dem ersten und dem zweiten Port regelt, um somit den Fluidstrom zwischen dem wenigstens einen Fluidbehälter und der darauf wirkenden Restriktionseinheit zu regulieren.

## Revendications

1. Système de restriction à auto-régulation, comprenant:
un dispositif de restriction (120) configuré pour recevoir du fluide et pour former une restriction dans un chemin correspondant à une quantité de fluide se trouvant dans celui-ci; et
un système de logique à fluide (130) couplé au dispositif de restriction et configuré pour réguler une quantité de fluide dans le dispositif de restriction en réponse à une pression de fluide agissant sur celui-ci, où le système de logique à fluide comporte au moins un réservoir de fluide (140, 150) et au moins une vanne couplée entre au moins un réservoir de fluide précité et le dispositif de restriction;
**caractérisé en ce que** la au moins une vanne comprend une vanne logique (132), où la vanne logique est configurée pour réguler l'écoulement du fluide en réponse à une pression du fluide dans le dispositif de restriction agissant sur celle-ci;
comprenant en outre un mécanisme de sollicitation (144) couplé à la vanne logique et apte à appliquer une force de sollicitation à la vanne logique qui agit contre une force appliquée à la vanne logique par une pression du fluide dans le dispositif de restriction.

2. Système selon la revendication 1, dans lequel le système de logique à fluide définit au moins une limite de pression préréglée, et lorsqu'une pression du fluide dans le dispositif de restriction est inférieure ou supérieure à la au moins une limite de pression préréglée, la au moins une vanne se déplace d'une position fermée à une position ouverte pour permettre l'écoulement du fluide entre le au moins un réservoir de fluide et le dispositif de restriction.

3. Système selon la revendication 2, dans lequel le au moins un réservoir de fluide sous pression comprend un réservoir de fluide sous pression élevée (140) en communication fluidique avec le dispositif de restriction, et la au moins une limite de pression préréglée comprend une limite de pression préréglée minimale, et où lorsque la pression du fluide dans le dispositif de restriction est inférieure à la limite de pression minimale préréglée, la au moins une vanne est configurée pour s'ouvrir pour permettre l'écoulement du fluide du réservoir de fluide sous haute pression dans le dispositif de restriction.

4. Système selon la revendication 2, dans lequel le au moins un réservoir de fluide sous pression comprend un réservoir de fluide sous basse pression (150) en communication fluidique avec le dispositif de restriction, et la au moins une limite de pression préréglée comprend une limite de pression préréglée maximale, et où lorsque la pression du fluide dans le dispositif de restriction est supérieure à la limite de pression préréglée maximale, la au moins une vanne est configurée pour s'ouvrir pour permettre l'écoulement du fluide du dispositif de restriction dans le réservoir de fluide sous basse pression.

5. Système selon la revendication 2, dans lequel la limite de pression préréglée comprend une limite de pression préréglée maximale et une limite de pression préréglée minimale, et où la au moins une vanne est configurée pour s'ouvrir pour libérer le fluide dudit au moins un réservoir dans le dispositif de restriction lorsque la pression du fluide dans le dispositif de restriction est inférieure à la limite de pression préréglée minimale, et la au moins une vanne est configurée pour s'ouvrir pour libérer le fluide du dispositif de restriction dans le au moins un réservoir lorsque la pression du fluide dans le dispositif de restriction est plus grande que la limite de pression préréglée maximale.

6. Système selon la revendication 5, dans lequel le au moins un réservoir de fluide sous pression comprend un réservoir de fluide sous haute pression en communication fluidique avec le dispositif de restriction, et un réservoir de fluide à basse pression en communication fluidique avec le dispositif de restriction, et la au moins une vanne est configurée pour libérer le fluide du réservoir de fluide de haute pression et dans le dispositif de restriction et pour libérer le fluide du dispositif de restriction dans le réservoir de fluide à basse pression.

7. Système selon la revendication 2, dans lequel la au moins une limite de pression préréglée est ajustable.

8. Système selon la revendication 7, dans lequel la au moins une limite de pression préréglée est ajustable en ajustant une pression de fluide dans le au moins un réservoir de fluide.

9. Système selon la revendication 7, dans lequel la au moins une limite de pression préréglée est ajustable en ajustant un paramètre de la au moins une vanne.

10. Système selon la revendication 1, dans lequel le mécanisme de sollicitation est ajustable pour permettre l'ajustement de la force de sollicitation.

11. Système selon la revendication 1, dans lequel la vanne logique comprend un premier orifice (134) en communication fluidique avec le au moins un réservoir de fluide, un deuxième orifice (136) en communication fluidique avec le dispositif de restriction, et au moins un joint configuré pour réguler l'écoulement de fluide entre les premier et deuxième orifices pour réguler ainsi l'écoulement du fluide entre le au moins un réservoir de fluide et le dispositif de restriction agissant sur celui-ci.
